# EUROPEAN PATENT APPLICATION

(11) **EP 4 245 776 A1**
(43) Date of publication of application: **20.09.2023**
(21) Application number: 21891261.6
(22) Date of filing: 15.11.2021
(51) Int. Cl.: C07K 19/00, C12N 15/62, C12P 21/02, A61K 38/20, A61K 39/395, A61K 47/64, A61P 35/00, A61P 7/10

(54) **FUSION PROTEIN OF HUMAN SERUM ALBUMIN AND INTERLEUKIN-2, AND USE THEREOF**

(30) Priority: 16.11.2020 CN 202011280409
(71) Applicant: Tianjin Sinobiotech Ltd., Tiajin 300457 (CN); Fortunerock (China) Co., Ltd., Beijing 100026 (CN); Beijing Bio-Fortune, Ltd., Beijing 100026 (CN)
(72) Inventor: YU, Zailin, Beijing 100026 (CN); FU, Yan, Beijing 100026 (CN); YANG, Xiaonan, Tianjin 300457 (CN); FU, Yusong, Tianjin 300457 (CN); QIAO, Guoqiang, Beijing 100026 (CN); HOU, Qiong, Tianjin 300457 (CN); ZHU, Tao, Tianjin 300457 (CN)
(74) Representative: Zwicker, Jörk
(86) International application number: PCT/CN2021/130743
(87) International publication number: WO 2022/100741

(57) **Abstract**

Provided is a fusion protein of human serum albumin or a wild type thereof and human interleukin-2 or a mutation thereof, said fusion protein has a significantly extended *in vivo* half-life relative to recombinant interleukin-2, can be used alone for treating a tumor, and improves anti-tumor efficacy of an anti-PD-1 antibody or an anti PD-L1-antibody.

## Description

### Technical Field

The present invention relates to a fusion protein of human serum albumin, particularly wild-type human serum albumin, and human interleukin-2, particularly a mutant human interleukin-2. The fusion protein has a significantly extended *in vivo* half-life relative to recombinant interleukin-2, and thus can be used alone for treating a tumor. Moreover, the fusion protein can significantly improve the anti-tumor efficacy of an anti-PD-1 antibody or an anti-PD-L1 antibody.

### Background Art

(I) Human serum albumin (HSA) is a soluble monomeric protein that makes up half of the total proteins in the blood. As a basic carrier, albumin carries and delivers fatty acids, steroids, hormone molecules, etc., and its stable inert nature is an important factor in maintaining blood pressure. Serum albumin is a globular, non-glycosylated, 585-amino acid serum protein with a molecular weight of 65 kilodaltons. This protein (albumin precursor) is then transformed and processed by the Golgi apparatus to remove the guide polypeptide, and secreted outside the cell. Serum albumin has 35 cysteines, and in the blood, albumin is a monomer with 17 disulfide bonds (see Brown JR, "Albumin structure, Function, and Uses" Pergamon, N.Y., 1977). When the polypeptide is not secreted, the albumin product within the yeast cell is in a mismatched state, loses 90% of its antigenicity (compared to native albumin in plasma), and forms insoluble albumin aggregates, thus not having the biological activity of albumin. Albumin products currently used in clinical practice are all extracted from human plasma. The production of recombinant expressed albumin (rHSA) using microorganisms, especially *Pichia* engineering strains, has been disclosed in China granted patent ZL2004010057313.7 to YU, Zailin and FU, Yan.
   Albumin is the main component of blood, its content in the human body is 40 grams per liter of blood, and its half-life is 20 days. Albumin is also often used as a stabilizer in pharmaceutical preparations, especially in the manufacture of biopharmaceuticals and vaccines. In summary, after human serum albumin and a therapeutic protein are genetically fused in yeast or vertebrate cells to express as a recombinant fusion protein by the use of genetic engineering technology, the fusion protein will have a great advantage to resist enzymolysis in living organisms, and can greatly improve the life of the therapeutic protein *in vivo* and *in vitro*, in other words, it can increase the stability and half-life of the therapeutic protein in serum and during storage, achieving long-acting effects of the protein drug, greatly reducing the administration frequency, and obtaining better therapeutic effects in the clinical treatment of major diseases (YU, Zailin and FU, Yan, A Better Bioinnovative Drug-Long-Acting Recombinant Human Serum Albumin Fusion Protein, Chin Med Biotechnol, 2017, 12(3): 248-264).
(II) Human interleukin-2 (IL-2) is a natural cytokine, and was first discovered in 1976 by Doris A. Morgan *et al.* IL-2 is produced by activated T lymphocytes (helper T cells) in the antigen or mitotic stimulation, has immunomodulatory activity, can directly enhance and up-regulate the body's immune system, and can also up-regulate and enhance the antibody-dependent immune regulation or humoral (cell) immune response. IL-2 is one of the critical cytokines in the human body. IL-2 has four antiparallel, amphiphilic alpha helices that form a quaternary structure essential for its function. IL-2 acts through three different receptors: IL-2 receptor alpha (IL-2Rα; CD25), which has the functions of significantly stimulating and enhancing cytokine production and rapid release *in vivo*, and is also the key cytokine that leads to cytokine storm and immune overreaction when the enhancement is excessive; and IL-2 receptor beta (IL-2Rβ; CD122) and interleukin-2 receptor gamma (IL-2Rγ; CD132), which play a critical role in IL-2 signaling, particularly in inhibiting tumor cell growth. IL-2 is fused directly to either end of human serum albumin, subjected to amino acid substitution (mutation), and then forms a fusion protein with albumin, achieving unexpected long-acting effects, avoiding the formation of cytokine storm, enhancing or not reducing its ability to bind to IL-2Rβ/γ, and producing new biological functions such as inhibiting the growth of tumor cells, which can obtain significant new clinical applications (efficacies) in the clinical treatment of tumors.

Recombinant human interleukin-2, also known as Aldesleukin (Proleukin), is an effective anti-cancer drug. Its blood half-life is only about 3.5 h, and frequent blood administration is required in clinical practice, which is not conducive to patient compliance and clinical efficacy. Marketed drugs of recombinant interleukin-2 interact with high-affinity IL-2 receptors expressed on the surface of immune cells to stimulate the release of large amounts of cytokines, including various interferons, interleukins, tumor necrosis factors (TNFs) and other cytokines, and to induce the proliferation and differentiation of a variety of cells, including B and T cells, monocytes, macrophages, natural killer (NK) cells, cytotoxic T cells, tumor infiltrating lymphocytes (TILs) and lymphokine activated killer (LAK) cells. The anti-tumor activity of IL-2 is thought to be the result of the activation of cytotoxic lymphocytes. IL-2 drugs increase the levels of interleukin-1, tumor necrosis factor alpha/beta, interferon gamma and interleukin-6. IL-2 acts on immune cells by binding to cell surface receptors, and the number and nature of IL-2-receptor interactions determine the magnitude of the effect on the cells. Natural IL-2 can activate beta and gamma receptor cells to exert anti-tumor activity, but it will preferentially bind to the alpha receptor (CD25) on immune cells, which will cause great side effects, such as severe toxicity and immunosuppression, resulting in the formation of cytokine storm. rIL-2 has significant toxic side effects, including life-threatening and sometimes fatal vascular leak syndrome (VLS), and its half-life is short, requiring administration three times a day for more than eight days, which limit its clinical use. Clinically, patients have a significant clinical need for long-acting drugs.

To date, for all approved IL-2 therapies, including aldesleukin, CAR-T has been characterized by preferential activation of non-target cells, which is also the reason why their clinical applications are greatly limited.

IL-2 immunotherapy has been demonstrated in various clinical trials. IL-2 can be used to inhibit the development of lung and liver metastases of different immunogenic and non-immunogenic mouse cancers, alone or in combination with LAKs. Early studies in patients with advanced cancers have shown that high-dose IL-2 therapy, alone or in combination with LAKs, can mediate complete or partial regression of the cancers.

In order to improve the serious cytokine storm, toxic side effects and immune agitation caused by the binding of IL-2 to IL-2 receptor alpha, scientists have carried out a variety of innovative research and development, such as IL-2-like drugs NKTR-214, ALKS4230, THOR-707, etc., the research and development process of which can be described as twists and turns and is not stable.
1. The new drug, NKTR-214, developed by Nektar, is a CD122-biased cytokine agonist, which is conjugated to polyethylene glycol (PEG), and is designed to provide continuous signaling through the heterodimeric IL-2 receptor pathway (IL-2R) to preferentially activate and expand CD8+ T and NK effector cells by regulatory T cells (Tregs). The trial with NKTR-214 alone failed. The Plainview report has shown that NKTR-214 monotherapy shows an overall response rate (ORR) of 0% (0/28) in the EXCEL trial compared with that of previous IL-2 monotherapies of 15-29%. Lymphocytes need to be increased by 200-300% to achieve clinical efficacy, but NKTR-214 can only increase lymphocytes by 33-50%, and its therapeutic effect is too weak.
2. ALKS4230 is another interleukin-2-like drug developed by Alkermes, an engineered fusion protein consisting of a circularly arranged IL-2 and IL-2 receptor (IL-2R) to selectively activate medium-affinity IL-2R. The selectivity of ALKS4230 aims to overcome its limitations while taking full advantage of the anti-tumor effects of IL-2 therapy. Initial data from the phase I study have shown a unique mechanism of ALKS4230, with dose-dependent pharmacodynamic effects on circulating natural killer cells and CD8+ T cells, and minimal and dose-independent effects on immunosuppressive regulatory T cells.
   Based on data from the initial single-dose escalation cohort, the study of ALKS4230 at a dose of 3 µg/kg/day was launched in September 2018. In the ALKS4230 monotherapy, Alkermes will also continue to increase the dose for further study. At present, the application of ALKS4230 has not been successful.
3. Synthorx is developing THOR-707, also an IL-2 "non-alpha" Synthorin, for the treatment of solid tumors. Analysis of the effects of THOR-707 in a syngeneic mouse tumor model has shown that THOR-707, alone and in combination with PD-1 inhibitors, induced tumor infiltration of CD8+ T effector cells, resulting in anti-tumor effects. In preclinical studies, THOR-707 induced molecular markers of T cell activation and peripheral NK and CD8+ effector T cell proliferation *in vivo.* Synthorx expected to begin Phase I clinical trials with THOR-707 in 2019.
4. Proleukin's Aldesleukin (rhIL-2), a drug for the treatment of adult metastatic renal cell carcinoma and adult metastatic melanoma, has been used as a treatment of last resort for cancer patients when no other treatment option is available.
5. IL-2 amino acid site mutations. Oliver AST et al. (US2018/0142037) proposed to introduce a triple mutation F42A/Y45A/L72G at amino acid residue positions 42, 45 and 72 of the IL-2 to reduce its affinity for the receptor IL-2Rα. Aron M.Levin et al. (Nature, Vol 484, p 529-533, DOl: 10. 10 38/nature 10975) proposed an IL-2 mutant, IL-2 H9, called "superkine", which comprises quintuple mutation L80F/R81D/L85V/I86V/I92F, with enhanced binding to IL-2Rβ, thus enhancing stimulation of CD25- cells. Rodrigo Vazquez-Lombardi et al. (Nature Communications, 8: 15373, DOI:10.1038/ncomms15373) proposed a triple mutant human IL-2 protein IL-2 3X, which has the residue mutations R38D/K43E/E61R at amino acid residue positions 38, 43 and 61, respectively, resulting in non-binding of the mutant protein to IL-2Rα, in order to eliminate the activation bias of IL-2 for CD25+ cells. However, the activation bias of IL-2 3X for CD25+ cells still exists; the half-life of the mutant protein is very short, and it is difficult to investigate the druggability in clinical practice; and its expression level is low, and it is also not conducive to the subsequent large-scale production as a drug.

Nonetheless, the above PEG modification and a small amount of substitutions or mutations of IL-2 amino acid sequence all failed to well improve the IL-2 cytokine toxicity, or to solve the stability problem during the production and manufacture, especially suffering from a variety of problems as regards to the production cost. For example, in 2019, NKRT-214, an IL-2 product modified with 6 PEGs, created serious unrepeatable manufacturing stability problems for product batch production. The news broke caused the stock price of Narkert Thereaputic to plummet by more than 40%, indicating that the drug manufacturing process (CMC) is also the most critical factor to ensure the stability of drug quality.

### (III) Anti-PD-1/L1 antibody drugs

The full name of the protein PD-1 is programmed death receptor-1, with an English name of programmed cell death-1. The protein is an important immunosuppressive molecule and a member of the CD28 superfamily, which is located on the surface of T cells. The full name of the protein PD-L1 is programmed death receptor-ligand 1. The protein is a type I transmembrane protein with a size of 40 kDa, which is located on the surface of tumor cells. When a T cell encounters a tumor cell, PD-1 can bind to PD-L1, preventing the T cell from recognizing the tumor cell, and leading to the death of the T cell (programma deathy). The use of PD-1 inhibitors (e.g., specific antibodies against the PD-1 protein) can block the binding of the PD-L1 protein to the PD-1 receptor, allowing T cells to normally perform their tumor-killing effects. The use of PD-L1 inhibitors (e.g., antibodies specific for the PD-L1 protein) can block the binding of the PD-L1 protein on tumor cells to the PD-1 receptor on T cells, and can also allow T cells to normally perform their tumor-killing effects.

Anti-PD-L1/PD-1 antibodies are immune checkpoint blocking drugs that help T cells uncover the hypocrisy of tumor cells and restore their recognition and killing of tumor cells. There are currently five main approved PD-1/PD-L1 drugs (PD-1 drugs: Keytruda and Opdivo, and PD-L1 drugs: Tecentriq, Imfinzi and Bavencio). Domestic anti-PD-1/L1 antibodies include mainly Toripalimab, Sintilimab, Camrelizumab, etc. The true clinical efficacy of anti-PD-1/L1 monoclonal antibodies is only 15-30%, and there are significant side effects. There is an actual and particularly urgent demand for improving clinical efficacies.

The present invention takes the existing problems encountered by IL-2 in clinical practice that affect the clinical efficacy, and interleukin-2 analogs, variant amino acid substitutions and other products currently under research and development, and the shortcomings of the poor clinical efficacy of anti-PD-1/L1 antibody drugs as the technical innovation, and takes advantage of drug molecular structure innovation, hoping to obtain a product that can be administered to tumor patients at least once every two weeks to greatly improve the patient compliance, and the production cost of which is only 1/300 of Netkar's PEGylated interleukin-2, while solving the shortcomings of wild-type human interleukin-2. The drug molecular structure of the invented recombinant human serum albumin/interleukin-2 fusion protein and its analog fusion proteins determines that they do not bind to IL-2 receptor alpha (CD25), significantly reducing immune overactive toxic side effects such as the production of cytokine storm, and greatly reducing the patient's treatment costs, thus obtaining an innovative drug structure. The analog fusion proteins of the present invention can also be purposefully artificially designed and mutated by amino acid substitutions in terms of binding to interleukin-2 beta and gamma receptors, thus improving the ability to inhibit tumor growth. In particular, when used in combination with anti-PD-1/L1 antibody drugs, they can improve the tumor inhibition function of the anti-PD-1/L1 antibodies, improve the clinical efficacy, and inhibit the proliferation of tumors. The present invention will enable more patients who are in urgent need for treatment and whose medication is restricted by compliance and treatment costs to receive the necessary treatment, and unexpected technical advantages and inventiveness are obtained.

### Summary of the Invention

The present invention relates to a fusion protein of human serum albumin and human interleukin-2.

In one aspect, the present invention provides a fusion protein, comprising a human serum albumin mature peptide and a human interleukin-2 mature peptide. In one embodiment, the human serum albumin mature peptide and the human interleukin-2 mature peptide are linked directly. In one embodiment, the human serum albumin mature peptide and the human interleukin-2 mature peptide are linked indirectly via a peptide linker. In one embodiment, the human interleukin-2 mature peptide is fused to the N-terminus of the human serum albumin mature peptide at its C-terminus. In one embodiment, the human interleukin-2 mature peptide is fused to the C-terminus of the human serum albumin mature peptide at its N-terminus. In one embodiment, the human serum albumin mature peptide has a wild-type human serum albumin mature peptide sequence, such as as set forth in SEQ ID NO. 9. In one embodiment, the human serum albumin mature peptide has a mutant human serum albumin mature peptide sequence. In one embodiment, the human interleukin-2 mature peptide has a wild-type human interleukin-2 mature peptide sequence, such as as set forth in SEQ ID NO. 10. In one embodiment, the human interleukin-2 mature peptide has a mutant human interleukin-2 mature peptide sequence, for example comprising one or more of the following substitutions relative to the wild-type human interleukin-2 mature peptide sequence: T3A, R38A, F42A, Y45A, E62A, L72G and C 125A. In one embodiment, the mutant human interleukin-2 mature peptide sequence comprises substitutions T3A, F42A, Y45A, L72G and C125A relative to the wild-type human interleukin-2 mature peptide sequence as set forth in SEQ ID NO. 10. In one embodiment, the mutant human interleukin-2 mature peptide sequence comprises substitutions T3A, R38A, F42A, Y45A, E62A, L72G and C125A relative to the wild-type human interleukin-2 mature peptide sequence as set forth in SEQ ID NO. 10. In one embodiment, a mutant human interleukin-2 mature peptide comprising substitutions T3A, F42A, Y45A, L72G and C125A relative to wild-type human interleukin-2 mature peptide is linked directly to the N-terminus of the human serum albumin mature peptide at its C-terminus. In one embodiment, a mutant human interleukin-2 mature peptide comprising substitutions T3A, R38A, F42A, Y45A, E62A, L72G and C125A relative to the wild-type human interleukin-2 mature peptide is linked directly to the C-terminus of the human serum albumin mature peptide at its N-terminus. In one embodiment, the fusion protein has an amino acid sequence selected from the group consisting of SEQ ID NO. 1, SEQ ID NO. 3, SEQ ID NO. 5 or SEQ ID NO. 7.

In one embodiment, the fusion protein has one or more of the following properties:
(a) it has reduced binding to human IL-2 receptor alpha (CD25) as compared to wild-type human interleukin-2;
(b) it does not bind to the human IL-2 receptor alpha (CD25);
(c) it has enhanced binding to IL-2 receptor beta (CD122) and IL-2 receptor gamma (CD132) as compared to wild-type human interleukin-2;
(d) it has an *in vivo* half-life that is extended at least 1-fold, at least 2-fold, at least 5-fold, at least 10-fold, at least 15-fold, at least 20-fold or more as compared to the wild-type human interleukin-2 or an amino acid sequence variant thereof;
(e) it has reduced cytotoxicity as compared to the wild-type human interleukin-2;
(f) it has increased activity in inhibiting tumor growth as compared to the wild-type human interleukin-2; and/or
(g) it increases the activity of an anti-PD-1 antibody or an anti-PD-L1 antibody in inhibiting tumor growth by at least 1-fold, at least 2-fold, at least 5-fold, at least 10-fold, at least 15-fold, at least 20-fold or more.

In one embodiment, the fusion protein is used as a medicament. In one embodiment, the fusion protein is used for treating a tumor. In one embodiment, the fusion protein is used for treating a cancer. In one embodiment, the fusion protein is used for treating cancerous ascites. In one embodiment, the fusion protein is used alone. In one embodiment, the fusion protein is used in combination with an anti-PD-1 antibody or an anti-PD-L 1 antibody.

In one aspect, the present invention provides a nucleic acid encoding the fusion protein of the present invention. In one embodiment, the nucleic acid has a nucleotide sequence selected from the group consisting of SEQ ID NO. 2, SEQ ID NO. 4, SEQ ID NO. 6 or SEQ ID NO. 8.

In one aspect, the present invention provides a vector, comprising the nucleic acid of the present invention. In one embodiment, the vector is a cloning vector or an expression vector. In one embodiment, the vector is a plasmid vector or a viral vector.

In one aspect, the present invention provides a host cell, comprising the nucleic acid of the present invention, or comprising the vector of the present invention. In one embodiment, the host cell is a bacterial cell, a fungal cell, a plant cell or an animal cell. In one embodiment, the host cell is an *E. coli* cell, a *Pichia pastoris* cell or a CHO cell.

In one aspect, the present invention provides a method of producing the fusion protein of the present invention, comprising culturing the host cell of the present invention, such that the fusion protein is produced. In one embodiment, the method further comprises isolating and/or purifying the fusion protein.

In one aspect, the present invention provides a pharmaceutical composition, comprising the fusion protein of the present invention.

In one aspect, the present invention provides the use of the fusion protein of the present invention in the preparation of a medicament for the treatment of a tumor. In one aspect, the present invention provides the use of the fusion protein of the present invention in the preparation of a medicament for the treatment of a cancer. In one aspect, the present invention provides the use of the fusion protein of the present invention in the preparation of a medicament for the treatment of cancerous ascites. In one embodiment, the medicament is used alone. In one embodiment, the medicament is used in combination with an anti-PD-1 antibody or an anti-PD-L1 antibody.

In one aspect, the present invention provides a method of treating a tumor, comprising administering the fusion protein of the present invention to a subject having the tumor. In one aspect, the present invention provides a method of treating a cancer, comprising administering the fusion protein of the present invention to a subject having the cancer. In one aspect, the present invention provides a method of treating cancerous ascites, comprising administering the fusion protein of the present invention to a subject having cancerous ascites. In one embodiment, the method further comprises administering an anti-PD-1 antibody or an anti-PD-L1 antibody to the subject.

### Brief Description of the Drawings

Figure 1 shows results of the expression of the target proteins in the supernatant at different times after the fermentation of *Pichia* engineering strains for the recombinant human serum albumin/interleukin-2 fusion protein and its analog fusion proteins in a 500 L fermenter.
Figure 2 shows results of purification by Capto-MMC column chromatography of the recombinant human serum albumin/interleukin-2 fusion protein and its analog fusion proteins.
Figure 3 shows an SDS-PAGE protein electropherogram of purified stocks of the recombinant human serum albumin/interleukin-2 fusion protein and its analog fusion proteins under reducing and non-reducing conditions.
Figure 4 shows a purity profile of stocks of the recombinant human serum albumin/interleukin-2 fusion protein and its analog fusion proteins as measured by high performance liquid chromatography on gel (SEC-HPLC).
Figure 5 shows an SDS-PAGE electropherogram (A) and Western-blot (B) of the recombinant human serum albumin/interleukin-2 fusion protein and its analog fusion proteins, showing that the fusion proteins have a significant immune reaction with specific antibodies against human interleukin-2.
Figure 6 shows a graph of plasma concentration determination and half-life determination of the recombinant human serum albumin/interleukin-2 fusion protein and its analog fusion proteins in rats after subcutaneous administration.
Figure 7 shows relative changes in tumor volume between each administration group and the control group in the humanized PD-1 transgenic mouse tumor model after the administration of 9555 (molecular structure of the fusion protein: rIL-2v5/SA), one of the recombinant human serum albumin/interleukin-2 fusion protein and its analog fusion proteins.
Figure 8 shows a graph of efficacy results of the recombinant human serum albumin/interleukin-2 fusion protein analog 9555, alone or in combination with the anti-PD-1 antibody Keytruda, on days 2, 5 and 7 for the inhibition of the proliferation of mouse solid tumors.
Figure 9 shows relative changes in tumor volume between each administration group and the control group in the humanized PD-1 transgenic mouse tumor model after the administration of 9777 (molecular structure of the fusion protein: rHSA/IL-2v7), one of the recombinant human serum albumin/interleukin-2 fusion protein and its analog fusion proteins.

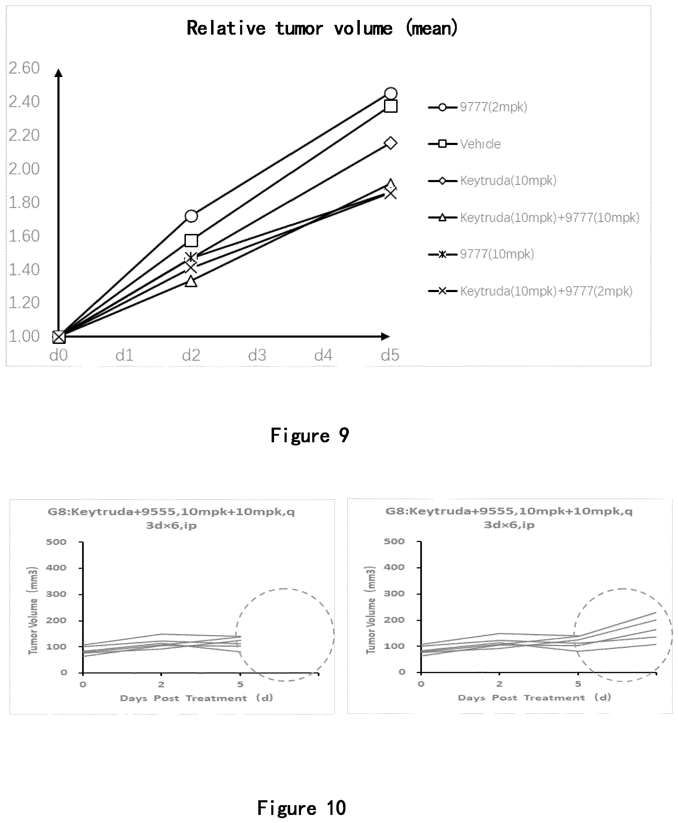

Figure 10 shows the tumor growth inhibitory effects between each administration group and the control group in the humanized PD-1 transgenic mouse tumor model after the administration of the recombinant human serum albumin/interleukin-2 fusion protein and its analog fusion proteins, especially the change of tumor proliferation after drug withdrawal. The left panel shows the tumor growth 5 days after administration (tumor growth is severely inhibited). The right panel shows the tumor growth in mice during days 5-8 after 3 days of drug withdrawal, showing that in the absence of drugs for inhibition, the tumors grow and develop rapidly again. The performance of the tumor growth with anti-tumor drugs for inhibition or drug withdrawal is consistent in all administration groups of mice.

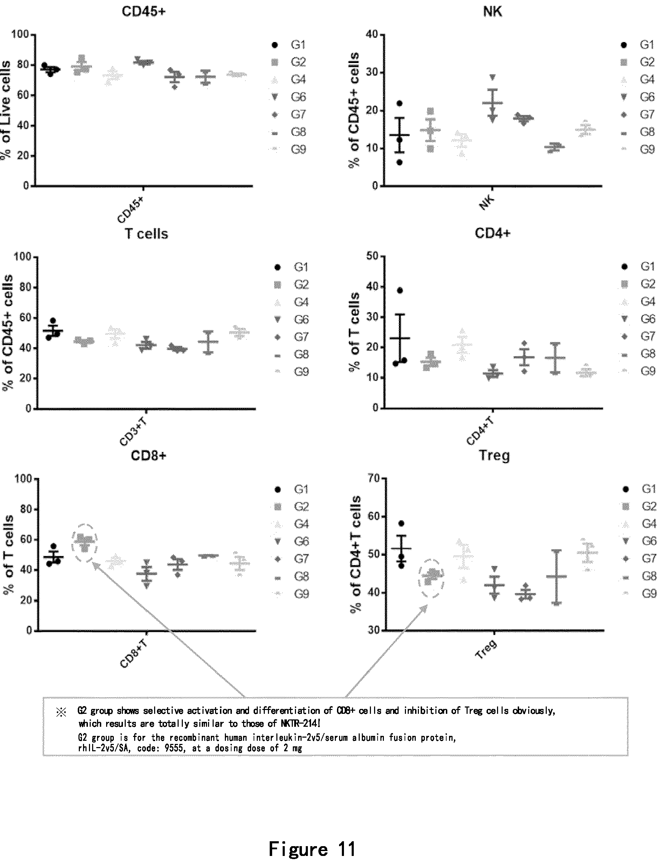

Figure 11 shows that the recombinant human serum albumin/interleukin-2 fusion protein and its analog fusion proteins of the present invention selectively activate and differentiate CD8+ cells and inhibit Treg cells obviously, which results are very similar to those of NKTR-214 (2019 ASCO). The G2 group (recombinant human interleukin-2v5/serum albumin fusion protein, rhIL-2v5/SA, code: 9555, in which G2 represents the dose group at a dosing dose of 2 mg) indicated by the dashed circle in Figure 11 shows a significant promotion effect on the proliferation of CD8+ cells, whereas it shows a tendency to significantly inhibit proliferation and decline in Treg cells.

### Detailed Description of Embodiments

The present invention is to solve the above-mentioned technical problems such as the short-acting effects of wild-type IL-2, the occurrence of serious "cytokine storm" toxic side effects in patients resulting from the binding of wild-type IL-2 to CD25 (IL-2 receptor alpha) in clinical practice, the non-significant effect of inhibiting tumor growth, etc., and proposes a novel molecular structure of a recombinant human serum albumin/interleukin-2 fusion protein and its analog fusion proteins, the use of the same as a medicament in clinical practice, and a method of preparing the same.

The present invention relates to the field of biotechnology, in particular a recombinant human serum albumin/interleukin-2 fusion protein and its analog fusion proteins having a long-acting anti-tumor effect significantly better than that of human interleukin-2, especially for malignant tumors such as adult metastatic renal cell carcinoma (RCC) and melanoma, as well as also suitable for being used as drugs for controlling cancerous hydrothorax and ascite. More specifically, the recombinant fusion protein and its analogs, when used in combination with anti-PD-1/L1 monoclonal antibody drugs, can increase the anti-tumor efficacy of the anti-PD-1/L1 antibody drugs by at least 1-fold or more. The present invention also discloses a method of preparing the recombinant human serum albumin/interleukin-2 fusion protein and its analog fusion proteins, and their use as a bioinnovative drug in clinical practice.

The recombinant human serum albumin/interleukin-2 fusion protein and its analog fusion proteins prepared by the present invention can be used as an innovative molecular structure for tumor treatment, and can be administered once at an interval of two weeks or more, with long-acting effects significantly better than the marketed recombinant interleukin-2 products. When used in combination with anti-PD-1/L1 monoclonal antibody drugs, the recombinant fusion protein and its analog fusion proteins have the ability to significantly reduce the binding to the receptor IL-2Rα, can significantly improve the anti-tumor efficacy of the anti-PD-1/L1 antibody drugs by at least 1-fold or more in inhibiting tumor growth, can significantly reduce the occurrence of cytokine storm, and have lower frequency of administration, better clinical efficacy and lower production cost.

The present invention is achieved according to the following technical solutions.

Provided are a recombinant human serum albumin/interleukin-2 fusion protein and its analog fusion proteins for treating a tumor, wherein the amino acid sequence of the recombinant human serum albumin/interleukin-2 fusion protein and its analog fusion proteins is SEQ ID NO. 1.

Further, the nucleic acid sequence of the recombinant human serum albumin/interleukin-2 fusion protein and its analog fusion proteins is SEQ ID NO. 2.

Provided are a recombinant human serum albumin/interleukin-2 fusion protein and its analog fusion proteins for treating a tumor, wherein the amino acid sequence of the recombinant human serum albumin/interleukin-2 fusion protein and its analog fusion proteins is SEQ ID NO. 3.

Further, the nucleic acid sequence of the recombinant human serum albumin/interleukin-2 fusion protein and its analog fusion proteins is SEQ ID NO. 4.

Provided are a recombinant human serum albumin/interleukin-2 fusion protein and its analog fusion proteins for treating a tumor, wherein the amino acid sequence of the recombinant human serum albumin/interleukin-2 fusion protein and its analog fusion proteins is SEQ ID NO. 5.

Further, the nucleic acid sequence of the recombinant human serum albumin/interleukin-2 fusion protein and its analog fusion proteins is SEQ ID NO. 6.

Provided are a recombinant human serum albumin/interleukin-2 fusion protein and its analog fusion proteins for treating a tumor, wherein the amino acid sequence of the recombinant human serum albumin/interleukin-2 fusion protein and its analog fusion proteins is SEQ ID NO. 7.

Further, the nucleic acid sequence of the recombinant human serum albumin/interleukin-2 fusion protein and its analog fusion proteins is SEQ ID NO. 8.

The recombinant human serum albumin/interleukin-2 fusion protein and its analog fusion proteins for tumor treatment are characterized in that relative to the wild-type human interleukin-2 fusion protein SEQ ID No. 3, the amino acid sequence of SEQ ID No. 5 has five specifically and site-specific substituted amino acids, namely T3A, F42A, Y45A, L72G and C125A, respectively.

The recombinant human serum albumin/interleukin-2 fusion protein and its analog fusion proteins for tumor treatment are characterized in that relative to the wild-type human interleukin-2 fusion protein SEQ ID No. 1, the amino acid sequence of SEQ ID No. 7 has seven specifically and site-specific substituted amino acids, namely T3A, R38A, F42A, Y45A, E62A, L72G and C125A, respectively.

In the recombinant human serum albumin/interleukin-2 fusion protein and its analog fusion proteins for tumor treatment, the amino acid sequence of an interleukin-2 mature peptide protein is fused directly to human albumin, wherein interleukin-2 is placed at different ends of an albumin mature peptide; and the interleukin-2 gene in the fusion proteins is substituted by specific and different amino acids, which result in reducing the ability to bind to the CD25 receptor (interleukin-2Rα), and improving the ability of the analog fusion proteins to bind to interleukin-2Rβ and Rγ (CD132/CD122), thus greatly improving the ability to inhibit tumor growth. The amino acid substitution of C125A in the interleukin-2 analog fusion protein gene can reduce the probability of the formation of dimers in the production of the interleukin-2 fusion proteins. In the preparation of all recombinant proteins, the formation of dimers is related directly to the occurrence of clinical immunogenicity (toxic side effects).

Further, the recombinant human serum albumin/interleukin-2 fusion protein and its analog fusion proteins for tumor treatment are characterized in that the C-terminus of the human interleukin-2 or variant analog mature peptide is linked directly to the N-terminus of human serum albumin (SEQ ID No. 3 or such as SEQ ID No. 5); or the N-terminus of the human interleukin-2 or variant analog mature peptide is linked directly to the C-terminus of human serum albumin (SEQ ID No. 1 or such as SEQ ID No. 7). The amino acid sequence of human serum albumin can be native or variant, or is a fragment of human albumin.

Further, the recombinant human serum albumin/interleukin-2 fusion protein and its analog fusion proteins for tumor treatment are characterized in that the human interleukin-2 analog fusion proteins do not bind or weakly bind to CD25 (also known as human interleukin-2 receptor alpha), which can significantly reduce the cytotoxicity of wild-type human interleukin-2 and improve the efficacy.

Further, the gene of the recombinant human serum albumin/interleukin-2 fusion protein and its analog fusion proteins is expressed by a recombinantly engineered host cell, wherein the recombinantly engineered host cell is a yeast, plant, bacterial or animal cell (e.g., an insect cell, such as a CHO cell); and the gene of the recombinant human serum albumin/interleukin-2 fusion protein and its analog fusion proteins is expressed through a plasmid DNA vector or a viral vector, or transferred to a plant and animal for expression via transgenic technology.

Further, the gene of the recombinant human serum albumin/interleukin-2 fusion protein and its analog fusion proteins is constructed and expressed through genetic engineering technology; and a method of obtaining the fusion proteins is to express the recombinant fusion proteins by means of transformation and transfection or infection using a vector plasmid.

Further, the gene of the recombinant human serum albumin/interleukin-2 fusion protein and its analog fusion proteins is transformed into *Pichia* strains by using a yeast expression vector, and the fusion proteins are secreted into the culture solution.

Further, the yeast host for the expression of the gene of the recombinant human serum albumin/interleukin-2 fusion protein and its analog fusion proteins is *Pichia pastoris* strain.

A method of high-density expressing and producing the above-mentioned recombinant human serum albumin/interleukin-2 fusion protein and its analog fusion proteins for the treatment of tumor indications comprises the following steps:
a. construction and expression of a vector plasmid for the recombinant human serum albumin/interleukin-2 fusion protein and its analog fusion proteins
   the HSA gene is synthesized and amplified by using the total RNA extracted from human fetal liver as the template through reverse transcription polymerase chain polymerization; the HSA DNA fragment is separated and purified by electrophoresis and cloned into a yeast expression vector to obtain a pYZ-HSA recombinant plasmid; the nucleotide sequence of human interleukin-2 or its analogs is artificially synthesized by DNA full sequence synthesis; the synthesized target gene (the gene of human interleukin-2 mutant analogs) and the pYZ-HSA recombinant plasmid are subjected to digestion, and linked together to obtain an expression recombinant plasmid for different pYZ-HSA/IL-2 fusion protein genes or their analog fusion proteins;
b. transformation and preparation of *Pichia* expression engineering strains
   the recombinant plasmid for the different pYZ-HSA/IL-2 gene fusions or the gene of their analog fusion proteins is treated with the *Pme*I restriction endonuclease and then point-transferred into *Pichia* X33 competent cells, and the transformed yeast strains are inoculated on YPD plate medium containing the Zeocin antibiotic for culture;
c. screening of recombinant yeast engineering strains
   several yeast colonies containing the gene to be expressed are each cultured in the basic culture solution containing the Zeocin antibiotic, a buffer and glycerol; after culturing for a period of time, the strains are collected by centrifugation, and resuspended in the same basic culture solution containing 0.5% methanol instead of glycerol; and 100% methanol is added every 24 hours to the final concentration of 0.5%, and the culture supernatant is collected at different time points to screen recombinant yeast engineering strains expressing the specific proteins; and
d. high-density expression and production of the recombinant human serum albumin/interleukin-2 fusion protein and its analog fusion proteins at a large scale
   500 L and 1000 L biological fermenter systems are used; a seed library of the engineering strains is cultured on a shaker at a small scale, then scaled up to a primary seed tank and a secondary seed tank, and allowed to enter a production fermenter; when the strains are cultured until glycerol in the tank is exhausted, and dissolved oxygen returns to the bottom limit and then rises again, the fed-batch addition of glycerol is initiated; when the fed-batch addition is over and the dissolved oxygen rises again, the fed-batch addition of methanol is initiated for 72 hours to enter the induction and recombinant protein production stage; and the yield is not less than 3 g/L of fermentation supernatant.

In a method of purifying the above-mentioned recombinant human serum albumin/interleukin-2 fusion protein and its analog fusion proteins for the treatment of tumor indications, the soluble recombinant human serum albumin/interleukin-2 fusion protein and its analog fusion proteins with a complete molecular structure, which are secreted directly into the fermentation supernatant after the high-density fermentation of the yeast engineering strains, can be prepared into pharmaceutical-grade active pharmaceutical ingredients (stocks) with a purity of not less than 95% through the following three-step enrichment, purification and production process. The purity of the target proteins in elution is high, and the recovery rate is considerable. This purification method varies depending on the molecular structure (difference in amino acid sequence) of the fusion proteins, but is simple and easy to implement.
a. Affinity chromatography column: The first step of separation, purification and enrichment of the target proteins is initiated by directly using the Capto-MMC packing medium from GE (US) through treating the fermentation supernatant of the yeast engineering strains for the recombinant human serum albumin/interleukin-2 fusion protein and its analog fusion proteins (codes are the molecular structures of different interleukin-2 fusion proteins) (see the Examples).
b. Hydrophobic medium chromatography column: Different recombinant human serum albumin/interleukin-2 fusion protein and its analog fusion proteins need to use different media for the purpose of separation, purification and enrichment in the second step. For example: octyl, butyl and phenyl media fillers (Outyl Sepharose^{™} High Performance, Butyl Sepharose^{™} High Performance or Phnyl High Performance, PFP) (see the Examples).
c. Purification, separation and chromatography column in Step 3: Different fusion proteins need to be further purified by using the same or different Q FF or Capto-DEAE fillers to obtain recombinant human serum albumin/interleukin-2 fusion protein and its analog fusion proteins, which meet the purity requirements of the stocks (active pharmaceutical ingredients) for clinical injection administration (see the Examples).

If necessary, the fusion proteins can be concentrated by conventional methods, chromatography (Sephacryl S-200 HR) or membrane dialysis to obtain high-concentration stocks, which are used for the production of finished products conforming to clinical medication.

The above-mentioned recombinant human serum albumin/interleukin-2 fusion protein and its analog fusion proteins can be prepared into a lyophilized powder injection dosage form, preferably a water injection dosage form, and further more preferably a pre-filled syringe administration dosage form.

The drug metabolism half-life of the above-mentioned recombinant human serum albumin/interleukin-2 fusion protein and its analog fusion proteins in animals and humans is 4-10 times the half-life of the finished recombinant human interleukin-2 products in the market, thus having long-acting effects.

Provided is the use of the above-mentioned recombinant human serum albumin/interleukin-2 fusion protein and its analog fusion proteins in the preparation of a medicament for the treatment of tumor indications, wherein the fusion proteins can reach an administration frequency of once every 2 weeks or less.

Provided is the use of the above-mentioned recombinant human serum albumin/interleukin-2 fusion protein and its analog fusion proteins in the preparation of a medicament for the clinical treatment of cancerous ascites.

When used in combination with an anti-PD-1 antibody, the above-mentioned recombinant human serum albumin/interleukin-2 fusion protein and its analog fusion proteins can significantly enhance the anti-tumor efficacy of the anti-PD-1 antibody by at least 1-fold, 2-fold, 5-fold, 10-fold or more.

When used in combination with an anti-PD-L1 antibody, the above-mentioned recombinant human serum albumin/interleukin-2 fusion protein and its analog fusion proteins can significantly enhance the anti-tumor efficacy of the anti-PD-L1 antibody.

The present invention achieves the following advantageous effects.

In the present invention, the C-terminus of the gene of the human interleukin-2 and its analog variant mature peptides is molecularly fused directly to the N-terminus of the human serum albumin gene, and no linker peptide is provided between them. In the present invention, the N-terminus of the gene of the human interleukin-2 and its analog variant mature peptides is molecularly fused directly to the C-terminus of the human serum albumin gene, and no linker peptide is provided between them. Through research and repeated tests, *Pichia* engineering strains for the expression of the long-acting recombinant human serum albumin/interleukin-2 fusion protein and its analog fusion proteins are constructed. A novel technique and method for the specific separation and purification of the recombinant human serum albumin/interleukin-2 fusion protein and its analog fusion proteins are established. The fusion proteins expressed by yeast have a complete molecular structure, and are secreted directly and soluble, which can be used in combination with antibodies that specifically recognize human serum albumin, or antibodies that specifically recognize human interleukin-2.

The nucleotide of the recombinant human serum albumin/interleukin-2 fusion protein and its analog fusion proteins of the present invention can be introduced into host cells by using recombinant gene cloning technology to allow the expression of the fusion proteins. The engineered host cells can be cultured in media containing conventional nutrients, and are subjected to appropriate modifications in order for the promoter to work efficiently. The culture conditions for selecting transformants or amplifying the nucleotide chain encoding the recombinant human serum albumin/interleukin-2 fusion protein and its analog fusion proteins are controlled in an appropriate manner of operation, such as temperature, pH and selection of high-expressing cell engineering strains.

The gene (nucleotide sequence) of the recombinant human serum albumin/interleukin-2 fusion protein and its analog fusion proteins of the present invention needs to be expressed by recombinantly engineered host cells. The recombinantly engineered host cells can be yeast strains, plant cells, bacteria, or animal cells (e.g., insect cells, such as CHO cells), and the fusion protein gene can be expressed directly by plasmid DNA vectors, can be expressed by viral vectors, or can be transferred to plants and animals for expression via transgenic technology.

The recombinant human serum albumin/interleukin-2 fusion protein and its analog fusion proteins mentioned in the present invention are preferably constructed and expressed using genetic engineering technology. A preferred method of obtaining the fusion proteins is to express the fusion proteins by means of transformation and transfection or infection using vector plasmids. It is particularly preferred to transform *Pichia* strains with transformable yeast expression vectors and to secrete the fusion proteins into the culture solution. A preferred yeast host for the expression of the recombinant human serum albumin/interleukin-2 fusion protein and its analog fusion proteins can be *Pichia pastoris* strain. The advantage of using yeast to express the recombinant human serum albumin/interleukin-2 fusion protein and its analog fusion proteins is that the yeast system can produce high-quality mature fusion proteins, which can be secreted into the culture solution to facilitate purification.

The progress of yeast genetic engineering has made it possible to express foreign genes in yeast and secrete protein products outside the cells. The advantages of using yeast to express secreted proteins are, but not limited to, high expression yields, acquisition of soluble proteins with a correctly folded protein structure, and ease of large-scale production and purification.

The present invention provides the use of yeast to produce the recombinant human serum albumin/interleukin-2 fusion protein and its analog fusion proteins more efficiently and cost-effectively. The present invention also provides and discloses by way of examples a ton-scale fermentation process of *Pichia* engineering strains expressing such high-yield, pyrogen-free recombinant human serum albumin/interleukin-2 fusion protein and its analog fusion proteins, and a method of separating and purifying the fusion proteins. The expression of the proteins after the fermentation of *Pichia* strains in a 1000 L fermenter is not less than 1 g/L, the purity after purification can reach 99%, and the endotoxin content is ≤ 0.5 EU/mg.

In the present invention, a composite novel sequential combination comprising weak cation exchange chromatography, hydrophobic column chromatography, ion exchange column chromatography and gel column chromatography is designed to remove impurities such as foreign proteins, carbohydrates, lipids, host proteins, etc., finally obtaining high-purity proteins.

In the present invention, the recombinant human serum albumin/interleukin-2 fusion protein and its analog fusion proteins are prepared by using a *Pichia* expression system. The recombinant protein drugs prepared by this system are completely suitable for clinical application, and the residual amount of host proteins in the stocks of the innovative drugs developed by the present invention also meets the requirements of the 2015 edition of Chinese Pharmacopoeia (part III).

In the present invention, the rapid test of high-dose protein drug water injection at 25°C is also completed to ensure that there is no problem with the stability of the novel dosage form (the long-term stability of 25°C for 6 months is equivalent to that the preparation can be stored stably for 3 years under the specified 2-8°C storage conditions).

The present invention enables the production cost of the injection or lyophilized powder injection (specification: 5 mg/ml) of the long-acting recombinant human serum albumin/interleukin-2 fusion protein and its analog fusion proteins produced after large-scale production to be between RMB 5 yuan to 10 yuan. At present, there are recombinant human interleukin-2 products in the market, however their production cost is high and the sales price is declining in the competition. The long-acting recombinant human serum albumin/interleukin-2 fusion protein and its analog fusion proteins of the present invention, which can be administered once every 2 weeks, are new generation upgrade products of human interleukin-2 drugs, with low production cost, large production capacity and less injection times, and can completely replace the existing short-acting and long-acting PEGylated human interleukin-2 and its analogs, variants and other products step by step. The technology of the present invention has great and obvious advantages in the market competition.

The recombinant human serum albumin/interleukin-2 fusion protein and its analog fusion proteins with different molecular structures specifically constructed in the present invention exhibit different biological functions in animal experiments, especially when used in combination with anti-PD-1 or anti-PD-L1 antibody drugs. Indeed, these unexpected new biological functions are likely to bring great potential clinical uses. Animal experiments have confirmed that fusion proteins of albumin and interleukin-2 with different molecular structures and different amino acid substitutions in the amino acid sequence of interleukin-2, when used alone to inhibit tumor growth or in combination with anti-PD-1 antibody drugs, exhibit significantly different tumor inhibition effects. The anti-tumor efficacy of the anti-PD-1 antibodies can be significantly enhanced by at least 1-fold, 2-fold, 5-fold, 10-fold or more.

The present invention is further illustrated in the drawings and the specific embodiments. However, these drawings and specific embodiments should not be considered as limiting the scope of the present invention.

### Examples

The present invention will be further described below with reference to the drawings and examples.

### Example 1: Expression of human serum albumin (HSA) gene and construction of vector plasmid

The HSA gene was synthesized and amplified by using the total RNA extracted from human fetal liver as the template through reverse transcription polymerase chain polymerization. The corresponding DNA strand was synthesized from 5 µg of total RNA under the action of reverse transcriptase (purchased from GIBCO/BRL). The reaction conditions were 45°C for 20 minutes, and then the temperature was raised to 55°C for an additional 40 minutes.

The DNA sequences of the oligonucleotide primers used were SEQ ID No. 11 and SEQ ID No. 12.

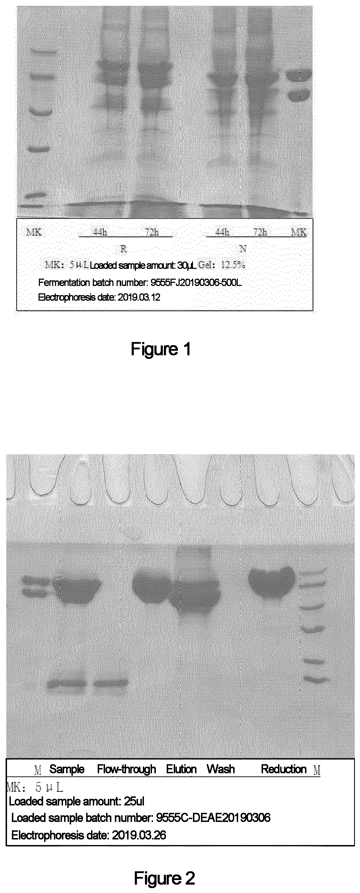

The HSA gene added with one *Eco*RI recognition site at each end was cloned into an expression vector. The PCR reaction conditions were 94°C for 4 minutes, and the DNA encoding HSA was further amplified for 35 cycles: 94°C, 30 sec; 58°C, 30 sec; and 72°C, 2 min 30 sec. At the end of the cycling reaction, an additional extension reaction was performed at 72°C for 10 minutes. Gel electrophoresis showed that the PCR amplification product was about 1850 base pairs in length. The PCR product was inserted into PCRII vector (Invitrogen) by TA cloning. The resulting plasmid was named PCR-HSA. DNA sequencing results showed that the HSA amino acid sequence translated therefrom was identical to the amino acid sequence of human serum albumin (GenBank, Access# V00494) published in GenBank. The PCR product was digested with EcoRI restriction enzyme, and the HSA DNA fragment was separated and purified by electrophoresis and cloned into a yeast expression vector (pYZ vector, purchased from TIANJIN SINOBIOTECH, LTD.). The expression vector was transformed into DH5a competent cells, positive single clones were screened on a low-salt LB-agar plate containing antibiotic Zeocin, and after identification, the plasmid into which HSA gene was inserted was named pYZ-HSA (see Figure 1, in which the pYZ-HSA recombinant plasmid was identified by digestion with *EcoR*I and *Xho*I, and the size of the target band was about 1850 bp, which was consistent with the expected size of the target band. Forward and reverse confirmation was performed by sequencing).

The amino acid sequence of the human serum albumin (HSA) mature peptide obtained by cloning was a sequence of SEQ ID No. 9, which was registered and preserved under ACCESS#: AY728024 in GeneBank by the inventors YU. Z/FU Y.

### Example 2: Expression of recombinant human serum albumin/interleukin-2 fusion protein and its analog fusion proteins and construction of vector plasmid

Based on GeneBank accession numbers NM_000586 and NP_000577.2, an amino acid sequence of SEQ ID No. 10 of the human interleukin -2 (wild type) mature peptide was obtained. The inventors designed the molecular structure of the recombinant human serum albumin fusion proteins by placing the interleukin-2 mature peptide at the C-terminus of the human serum albumin mature peptide in a "seamless connection" manner without a linker peptide therebetween, resulting in SEQ ID No. 1, to which the corresponding nucleotide sequence was SEQ ID No. 2; or placing the interleukin-2 mature peptide at the N-terminus of the human serum albumin mature peptide in a "seamless connection" manner without a linker peptide therebetween, resulting in SEQ ID No. 3, to which the corresponding nucleotide sequence was SEQ ID No. 4.

According to the known computer analysis and literature, as well as the molecular model of IL-2 binding to three receptors on the cell surface, after the inventors selected different point mutations and co-mutations in IL-2 through many experiments, and carried out the biological activity determination of the mutants at the cell level and the analysis of animal tumor models, the inventors designed the molecular structure of the recombinant human serum albumin fusion proteins by placing the interleukin-2 mature peptide mutant analog, which was subjected to amino acid substitution, at the N-terminus of the human serum albumin mature peptide in a "seamless connection" manner without a linker peptide therebetween, resulting in SEQ ID No. 5, to which the corresponding nucleotide sequence was SEQ ID No. 6. Compared with the wild-type IL-2 (corresponding to SEQ ID No. 10), the amino acid sequence of SEQ ID No. 5 had five substituted amino acids in total, namely T3A, F42A, Y45A, L72G and C125A, respectively.

The inventors also placed the interleukin-2 mature peptide at the C-terminus of the human serum albumin mature peptide in a "seamless connection" manner without a linker peptide therebetween, resulting in SEQ ID No. 7, to which the corresponding nucleotide sequence was SEQ ID No. 8. Compared with the wild-type IL-2 (corresponding to SEQ ID No. 10), the amino acid sequence of SEQ ID No. 7 had seven substituted amino acids in total, namely T3A, R38A, F42A, Y45A, E62A, L72G and C125A, respectively.

To this end, based on the amino acid sequence of the rhIL-2v5/SA mature peptide, the nucleotide sequence of the IL-2 mutant mature peptide can also be artificially synthesized by DNA full sequence synthesis. In artificial synthesis, *Xho*I cleavage site can be added to its N-terminus, and the human serum albumin mature peptide sequence can be added to *Ale*I cleavage site at its C-terminus. These two enzymes can be used to digest the synthesized target gene IL-2v5 and the HSA gene sequence in pYZ-HSA, respectively, forming a recombinant plasmid. The secretion of peptides can be completed through the secretion of a recombinant sequence of the target gene IL-2v5 linked to the HSA gene by virtue of the alpha-factor of yeast. The specific steps were as follows: The digested target gene IL-2v5 was mixed with the HSA recombinant DNA plasmid at a certain proportion, and they were allowed to ligate at 22°C for 1 hour after adding a ligation buffer and a ligase. The ligation product was transformed into DH5α competent cells, in which the specific steps were as follows: the ligation product was added to DH5α competent cells, and the cells were subjected to an ice bath for 30 minutes, heat shock at 42°C for 90 seconds, and an ice bath for another 2 minutes; the culture medium was added, and the mixture was placed in a shaker at 150 rpm for 45 minutes; and the culture was centrifuged at 12000 rpm for 1 minute, and the resultant was used for plating. Verification by sequencing was performed by the specific steps: single clones were picked and expanded, a small amount of plasmid was extracted, identification was performed by digestion, the plasmid having a digested fragment with the same size as the target fragment was selected, and verification by sequencing was performed using forward sequencing primer of SEQ ID No. 13 (P1) and reverse sequencing primer of SEQ ID No. 14 (P9). The final plasmid confirmed by full-length sequencing was named pYZ-rhIL-2v5/SA.

### Example 3: Transformation and preparation of Pichia pastoris expression engineering strains

Colonies of *Pichia pastoris* strain X33 were inoculated into a 50 ml centrifuge tube containing 5 ml of YPD culture solution, and cultured overnight at 30°C at 250 rpm. The following day, 0.2 ml of the overnight culture was inoculated into 500 ml of YPD culture solution in a 2 L triangular culture flask. Rotation culture was performed at 30°C for 2-3 hours, and the cell density reached OD₆₀₀ = 1.3-1.5. The yeast strains were collected by centrifugation, resuspended in 500 ml of ice-cold sterile water, and washed twice. The yeast strains were then suspended in 20 ml of ice-cold 1M sorbitol solution, and washed once.

Taking the pYZ-rhIL-2v5/SA plasmid DNA constructed in Example 2 as an example, the plasmid DNA was treated with restriction endonuclease *Pme*I, forming a linear plasmid molecule. 5 µg of linearized plasmid DNA was mixed with 80 µl of treated yeast strains in a 0.2 cm thick electrode cup, and the electrode cup was placed on an electroporator. The electric pulse conditions were a voltage of 7500 V/CM and an electric shock interval of 5-10 (ms). Immediately after the electric shock treatment, 1 ml of ice-cold 1M sorbitol solution was added to the yeast strains, and the mixture was then transferred to a 15 ml tube. The transformed yeast strains were placed in an incubator at 30°C for 2 hours, and then inoculated onto YPD plate medium containing antibiotic Zeocin. Clones grown after resistance selection were further identified for gene insertion using molecular biology methods. Protein expression and secretion were detected by SDS-PAGE or Western blot with specific antibodies.

### Example 4: Screening of recombinant yeast engineering strains

Several yeast colonies containing the gene to be expressed were each cultured in the basic culture solution containing antibiotic Zeocin, a buffer and glycerol. The colonies were cultured at 300 rpm, until the strain density reached OD₆₀₀ = 2-6. The strains were collected after centrifuging the culture at 1500 rpm for 15 minutes. The strains were resuspended in the same basic culture solution containing 0.5% methanol instead of glycerol. The cell density reached OD₆₀₀ = 1.0, and the culture was continued. Under the induction of methanol, the yeast strains began to express the foreign proteins under the action of the promoter. Thereafter, 100% methanol was added every 24 hours to a final concentration of 0.5%. The culture supernatant was collected at different time points. The expression of the recombinant human serum albumin/interleukin-2 fusion protein and its analog fusion proteins was preliminarily determined by SDS-PAGE (denaturing polyacrylamide gel electrophoresis), and the recombinant yeast engineering strains expressing the specific proteins of interest were screened.

### Example 5: High-density expression of recombinant human serum albumin/interleukin-2 fusion protein and its analog fusion proteins at large scale

In present invention, 500 L and 1000 L (ton-level) biological fermenter systems were used to carry out the ton-level large-scale production and fermentation process of the recombinant human serum albumin/interleukin-2 fusion protein and its analog fusion proteins, as well as the kilogram-level preparation process and technology establishment of the recombinant human serum albumin/interleukin-2 fusion protein and its analog fusion proteins, respectively. A seed library of the engineering strains was prepared according to the process of the research and development of the recombinant yeast engineering strains. The strains in a seed tube of the working seed library of the engineering strains were inoculated into a triangular flask, and the strains were cultured on a shaker at a small scale, then scaled up to a primary seed tank and a secondary seed tank, and allowed to enter a production fermenter (volume: 1 ton). When the strains were cultured until glycerol in the tank was exhausted, and dissolved oxygen returned to the bottom limit and then rose again, the fed-batch addition of glycerol was initiated. When the fed-batch addition was over, and the dissolved oxygen rose again, the fed-batch addition of methanol was initiated for 72 hours to enter the induction and recombinant protein production stage. Samples can be taken at different culture time points to test the expression of the recombinant proteins. The contents of secreted proteins in cells and the culture solution were both analyzed by SDS-PAGE, and the expression level and purity were monitored in each step. The results showed that the expression level of the rhIL-2/SA protein in the fermentation broth was about 3-10 g/L. Figure 1 shows results of the expression of the target proteins in the supernatant at different times after the fermentation of *Pichia* engineering strains for the recombinant human serum albumin/interleukin-2 fusion protein and its analog fusion proteins in a 500 L fermenter. In the figure, the lanes are as follows from left to right: Lane 1: in-house MK (molecular weights from top to bottom: 83 KD and 66 KD); Lane 2: reduced rhIL-2/SA 52 hours after the expression induction, with a molecular weight of ~81.6 kD; Lane 3: reduced rhIL-2/SA 72 hours after the expression induction, with a molecular weight of ~81.6 kD; Lane 4: non-reduced rhIL-2/SA 52 hours after the expression induction, with a molecular weight of ~81.6 kD; Lane 5: non-reduced rhIL-2/SA 72 hours after the expression induction, with a molecular weight of -88.6 kD; and Lane 6: proteins with standard molecular weights (from top to bottom: 97.4 kD, 66.2 kD, 43.0 kD, 31.0 kD, 20.1 kD and 14.4 kD). SDS-PAGE is divided into non-reducing SDS-PAGE and reducing SDS-PAGE, both of which are electrophoresis under denaturing conditions. Reducing and non-reducing SDS-PAGE are distinguished by the addition or absence of a reducing agent (e.g., DTT or 2-mercaptoethanol, etc.) during sample processing.

### Example 6: Purification and characterization of secreted recombinant human serum albumin/interleukin-2 fusion protein and its analog fusion proteins

The recombinant human serum albumin/interleukin-2 fusion protein and its analog fusion proteins were secreted directly into the supernatant by the fermentation of the yeast engineering strains. After separating the supernatant containing the secreted recombinant human serum albumin/interleukin-2 fusion protein and its analog fusion proteins from the strains by continuous flow centrifugation, the column chromatography purification was performed first by the composite novel weak cation exchange chromatography Capto-MMC of GE (US). Since the expressed supernatant had complex composition and contained a certain concentration of salt, and the Capto-MMC chromatography can withstand high flow rate and high loading capacity and its requirement for the salt concentration in the fermentation supernatant is not high, this chromatography was very suitable for the first step of column chromatography collection of the fermentation broth.

For the Capto-MMC chromatography, 10-100 mM NaAc-HAC buffer with a pH in the range of 4.5-6.5 can be selected, 10-100 mM phosphate buffer containing 0.1-0.5M NH₄Cl or other high salt solutions with a pH in the range of 4.5-8.5 can be used as the elution buffer, or various concentrations of organic solvents can also be used. A certain amount of amino acid (such as glycine or cysteine) can also be added to the buffer to maintain the stability of the fusion proteins.

After a large number of screening and exploratory experiments, the specific experimental flow can be as follows:

| **Fusion protein code** | **First step of purification Capto-MMC** |
|---|---|
| 9222 | 1. Protein source: 9222's fermentation supernatant, pH 5.0 |
| | 2. Purification filler: MMC, column volume: 7 L |
| | 3. Loaded sample amount and flow rate: loaded sample amount = 200 L, flow rate = 300 ml/min |
| | 4. Equilibration buffer: 25 mM NaAc, 2% glycine, 10 mM cysteine, pH 5.0 |
| | 5. Washing buffer: 50 mM PB, 2% glycine, 10 mM cysteine, pH 7.0, 0.05M NH₄Cl |
| | 6. Elution buffer: 50 mM PB, 2% glycine, 10 mM cysteine, pH 7.0, 0.6M NH₄Cl |
| 9333 | 1. Protein source: 9333's fermentation supernatant, pH 5.0 |
| | 2. Purification filler: MMC, column volume: 7 L |
| | 3. Loaded sample amount and flow rate: loaded sample amount = 200 L, flow rate = 300 ml/min |
| | 4. Equilibration buffer: 25 mM NaAc, 2% glycine, 10 mM cysteine, pH 5.0 |
| | 5. Washing buffer: 50 mM PB, 2% glycine, 10 mM cysteine, pH 7.0, 0.05M NH₄Cl |
| | 6. Elution buffer: 50 mM PB, 2% glycine, 10 mM cysteine, pH 7.0, 0.6M NH₄Cl |
| 9555 | 1. Protein source: 9555's fermentation supernatant |
| | 2. Purification filler: MMC, column volume: 7 L |
| | 3. Loaded sample amount and flow rate: loaded sample amount = 160 L, flow rate = 300 ml/min |
| | 4. Equilibration buffer: 25 mM NaAc buffer, pH 5.0, 2% glycine, 50 mM L-Arg + L-Glu |
| | 5. Washing buffer: 50 mM PB, pH7.0 |
| | 6. Elution buffer: 0.6M NH4Cl, 50 mM PB, pH 7.0, 2% glycine, 50 mM L-Arg + L-Glu |
| 9777 | 1. Protein source: 9777's fermentation supernatant, pH 5.0 |
| | 2. Purification filler: MMC, column volume: 7 L |
| | 3. Loaded sample amount and flow rate: loaded sample amount = 200 L, flow rate = 300 ml/min |
| | 4. Equilibration buffer: 25 mM NaAc, pH 5.0 |
| | 5. Washing buffer: 50 mM PB, pH 7.0 |
| | 6. Elution buffer: 0.6M NH4Cl, 50 mM PB buffer, pH 7.0, 2 mM EDTA, 2 mM EGTA |

For the peak fraction containing the target proteins preliminarily purified by Capto-MMC, a second step of the process that can directly perform the separation and purification with a hydrophobic media filler was specially explored for ease and effectiveness of the production process. To this end, the inventors continued to compare and screen hydrophobic fillers with butyl, phenyl or octyl groups. In addition, 20-100 mM Tris-HCl or phosphate buffer with a pH in the range of 5.0-9.0 was selected, the mentioned buffer with 1-2M NH₄Cl or 1-2M (NH₄)₂SO₄ added was used as the equilibration buffer, the mentioned buffer without NH₄Cl or (NH₄)₂SO₄ was used as the elution buffer, and a certain amount of glycine or cysteine can also be added to the buffer to maintain the stability of the proteins. The process included diluting the elution peak from the Capto-MMC chromatography column several times with 20-100 mM Tris-HCl or phosphate buffer containing 1-2M NH₄Cl or 1-2M (NH₄)₂SO₄ (pH 5.0-9.0), or directly adding a high concentration of NH₄Cl or (NH₄)₂SO₄ solution to the elution peak from the Capto-MMC chromatography column to a concentration of 1-2M NH₄Cl or 1-2M (NH₄)₂SO₄, loading the sample on a hydrophobic column, then washing the column with an equilibration buffer, and then eluting the column with an elution buffer to collect the elution peak. Possible impurities such as polymers, carbohydrates, lipids, degraded proteins, pigments, etc. can be further removed by a hydrophobic column to further improve the purity of the target proteins. Figure 2 shows results of purification by Capto-MMC column chromatography of the recombinant human serum albumin/interleukin-2 fusion protein and its analog fusion proteins. It is an SDS-PAGE electrophoretogram of each component peak sample after the fermentation supernatant was directly loaded onto the Capto-MMC chromatography column. In the figure, the bands from left to right are as follows: Lane 1. M, with molecular weights of 85 kD and 66 kD from top to bottom, respectively; Lane 2. fermentation supernatant component; Lane 3. supernatant flow-through component; Lane 4. non-reducing electrophoresis of the component eluted with the elution buffer; Lane 5. component eluted with the washing buffer; Lane 6. reduction electrophoresis of the elution component of Lane 4; and Lane 7. proteins with standard molecular weights (from top to bottom: 97 kD, 66 kD, 45 kD, 35 kD, 24 kD and 14 kD), respectively.

After a large number of screening and exploratory experiments, the flow of the second step of the further hydrophobic medium purification process was as follows:

| **Fusion protein code** | **Second step of purification** |
|---|---|
| 9222 | 1. Protein source: 9222's MMC purified sample |
| | 2. Purification filler: Butyl, column volume: 380 ml |
| | 3. Loaded sample amount and flow rate: loaded sample amount = 400 ml, flow rate = 15 ml/min |
| | 4. Equilibration buffer: 50 mM PB, 2% glycine, 10 mM cysteine, pH 7.0, 2M NH₄Cl |
| | 5. Elution buffer: 50 mM PB, 2% glycine, 10 mM cysteine, pH 7.0 |
| | 6. Elution volume: elution buffer = 100 ml, water = 100 ml |
| 9333 | 1. Protein source: 9333's MMC purified sample |
| | 2. Purification filler: PHP, column volume: 344 ml |
| | 3. Loaded sample amount and flow rate: loaded sample amount = 600 ml, flow rate = 20 ml/min |
| | 4. Equilibration buffer: 50 mM PB, pH 6.5, 1.2M NH4Cl, 2% glycine |
| | 5. Elution buffer A: 20 mM PB, pH 6.5, 0.2M NH₄Cl, 2% glycine |
| | 6. Elution buffer B: 20 mM PB, pH 6.5, 2% glycine |
| 9555 | 1. Protein source: 9555's MMC purified sample |
| | 2. Purification filler: Outyl, 700 ml |
| | 3. Loaded sample amount and flow rate: loaded sample amount = 2000 ml, |
| | flow rate = 50 ml/min |
| | 4. Equilibration buffer: 2.0M NH₄Cl, 50 mM PB, pH 6.5, 2% glycine, 50 mM L-Arg + L-Glu |
| | 5. Elution buffer: 50 mM PB, pH 6.5, 2% glycine, 50 mM L-Arg + L-Glu |
| | 6. Harvest volume: elution buffer = 340 ml, water = 290 ml |
| 9777 | 1. Protein source: 9777's MMC purified sample |
| | 2. Purification filler: Butyl, column volume: 1344ml |
| | 3. Loaded sample amount and flow rate: loaded sample amount = 1500 ml, flow rate = 45 ml/min |
| | 4. Equilibration buffer: 50 mM PB, pH 7.2, 1.0M NH₄Cl, 2 mM EDTA, 2 mM EGTA |
| | 5. Elution buffer: 50 mM PB, pH 7.2, 2 mM EDTA, 2 mM EGTA |

In view of the production process, the sample purified by hydrophobic chromatography was subjected to a third step of repurification by an anion exchange column such as Q or DEAE. Ion exchange chromatography allows for further purification of the target proteins by a separation mechanism different from hydrophobic chromatography. 20-100 mM Tris-HCl or phosphate buffer with a pH in the range between 6.0-9.0 can be selected as the equilibration buffer, and a certain amount of glycine or cysteine can also be added to the buffer to maintain the stability of the proteins. NaCl or NH₄Cl at a concentration of 200-1000 mM can be added to the equilibration buffer as the elution buffer. Samples subjected to anion exchange chromatography can usually reach a purity of 95% or more.

After a large number of screening experiments, the experimental flow of the third step of the purification process was as follows:

| **Fusion protein code** | **Third step of purification** |
|---|---|
| 9222 | 1. Protein source: 9222's Butyl sample |
| | 2. Purification filler: Q ff, column volume: 59 ml |
| | 3. Loaded sample amount and flow rate: loaded sample amount = 200 ml, flow rate = 15 ml/min |
| | 4. Equilibration buffer: 20 mM PB, 2% glycine, 10 mM cysteine, pH 6.5 |
| | 5. Elution buffer: 20 mM PB, 2% glycine, 10 mM cysteine, pH 6.5, 80 mM NaCl |
| 9333 | 1. Protein source: 9333's PHP sample |
| | 2. Purification filler: Q ff, column volume: 59 ml |
| | 3. Loaded sample amount and flow rate: loaded sample amount = 200 ml, flow rate = 15 ml/min |
| | 4. Equilibration buffer: 20 mM PB, 2% glycine, 10 mM cysteine, pH 6.5 |
| | 5. Elution buffer: 20 mM PB, 2% glycine, 10 mM cysteine, pH 6.5, 80 mM NaCl |
| 9555 | 1. Protein source: 9555020190306 |
| | 2. Purification filler: Capto-DEAE, column volume: 20 ml |
| | 3. Loaded sample amount and flow rate: loaded sample amount = 200 ml, flow rate = 4 ml/min |
| | 4. Equilibration buffer: Tris-HCl, pH 8.0, 2.0% glycine, 50 mM L-Arg + L-Glu |
| | 5. Elution buffer: 60 mM NaCl, Tris-HCl, pH 8.0, 2.0% glycine, 50 mM L-Arg + L-Glu |
| | 6. Washing buffer: 1M NaCl, Tris-HCl, pH 8.0 |
| 9777 | 1. Protein source: 9777's Butyl sample |
| | 2. Purification filler: Q ff, column volume: 59 ml |
| | 3. Loaded sample amount and flow rate: loaded sample amount = 200 ml, flow rate = 15 ml/min |
| | 4. Equilibration buffer: 20 mM PB, 2% glycine, 10 mM cysteine, pH 6.5 |
| | 5. Elution buffer: 20 mM PB, 2% glycine, 10 mM cysteine, pH 6.5, 120 mM NaCl |

The elution peak collected after ion exchange chromatography can be loaded directly on a gel chromatography column such as Sephacryl, Superdex or Sephadex for desalting and buffer exchange. In the gel chromatography, 5-50 mM phosphate buffer (PB) was used as the equilibration buffer, and a certain amount of glycine or cysteine can also be added to the buffer to maintain the stability of the proteins. The buffer system of the target protein rhIL-2/SA obtained under the conditions of anion exchange chromatography can be changed to 5-10 mM phosphate buffer, which can be used directly for the formulation of the preparation without using other means such as dialysis or ultrafiltration, so as to reduce possible pollution, and loss caused by protein denaturation, degradation and aggregation in the treatment steps.

After a large number of screening experiments, the flow of the concentration process was as follows:
Chromatography column: Sephacryl S-200 HR
Equilibration buffer: 20 mM PB, 5% glycine, pH 7.2

The whole purification process was reasonable in order, easy to operate, and easy to scale up in industrial production, and a single component in the final product can reach 99% (≥ 95% or more). If the polymers, dimers and related proteins of the target proteins were calculated as the target proteins, the residual amount of host proteins was less than 0.05%. In the present invention, a separation, purification and production process for producing the recombinant human serum albumin/interleukin-2 fusion protein and its analog fusion proteins at a large scale was developed, and a high purification effect with a final purity of 99% or more was obtained.

### Example 7: Determination of purity of recombinant human serum albumin-interleukin-2 fusion protein and its analog fusion proteins by high performance liquid chromatography

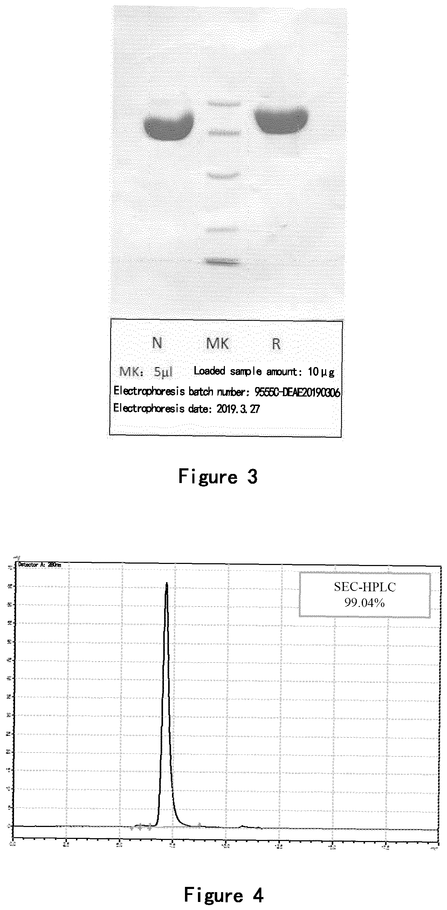

Shimadzu LC-lOAvp Plus high performance liquid chromatograph equipped with chromatographic column TSK-GEL G2000SWXL 7.8 × 300 mm (TOSOH) was used. 50 mM PB-0.1M NaCl buffer (pH 7.0) was used as the mobile phase, flow rate: 0.8 ml/min, detection wavelength: 280 nm. The recombinant human serum albumin/interleukin-2 fusion protein and its analog fusion proteins were obtained with a purity of 99.62% (SDS-PAGE) by the optimized separation and purification process established by the above procedures. Figure 3 shows an SDS-PAGE protein electropherogram of purified stocks of the recombinant human serum albumin/interleukin-2 fusion protein and its analog fusion proteins under reducing (R) and non-reducing (N) conditions. Figure 4 shows a purity profile of stocks of the recombinant human serum albumin/interleukin-2 fusion protein and its analog fusion proteins as measured by high performance liquid chromatography on gel (SEC-HPLC). The content of the target protein-related proteins (polymers and target protein degradation products) was less than 1%.

### Example 8: Detection of residual amount of yeast host proteins in recombinant human serum albumin/interleukin-2 fusion protein and its analog fusion proteins

By utilizing the detection method disclosed by the inventors in the Chinese invention patent (CN 109851674 A), the detection was performed with the detection kit Immunoenzymetric Assay Kit for the Measurement of *Pichia pastoris* Host Cell Proteins (Cygnus, US; Art.No.: Cat# F140), detection wavelength: 450/650 nm. Preparation of standard solution: the standard solution provided in the kit was used. Recovery rate determination wells (duplicated wells) were provided. The determination method was performed according to the operating instructions provided by the kit manufacturer. The results showed that the residual amount of host proteins in the stocks of the recombinant fusion proteins should be no more than 0.01% of the total protein. After detecting the high-purity injectable recombinant fusion protein products produced by the production process of the present invention, the results showed that the residual amount of host proteins was less than 0.01%. In other words, the purity of the target proteins had reached 99.99%. The yeast host proteins were the proteins, enzymes secreted by yeast produced during the fermentation of the yeast strains, and degraded host proteins produced by yeast. Host protein residues can enter the blood after subcutaneous injection of drugs, and stimulate the body to produce immune response or show high fever, chills and other signs. Therefore, the smaller the residual amount of host proteins, the better. After detecting the high-purity injectable products of the recombinant human serum albumin/interleukin-2 fusion protein and its analog fusion proteins produced by the production process of the present invention, the results showed that the residual amount of host proteins was less than 0.01%. In other words, the purity of the target proteins had reached 99.99%.

### Example 9: Analysis of recombinant human serum albumin/interleukin-2 fusion protein and its analog fusion proteins by Western blot

The purified recombinant human serum albumin/interleukin-2 fusion protein and its analog fusion proteins were subjected to SDS-PAGE electrophoresis. The proteins were transferred to a cellulose acetate membrane with an electrophoresis apparatus, and then specific antibodies (primary antibodies) were used to recognize the corresponding proteins. Then, secondary antibodies with horseradish peroxidase, which recognize and bind to the primary antibodies, left a blot on the cellulose acetate membrane after DAB development.

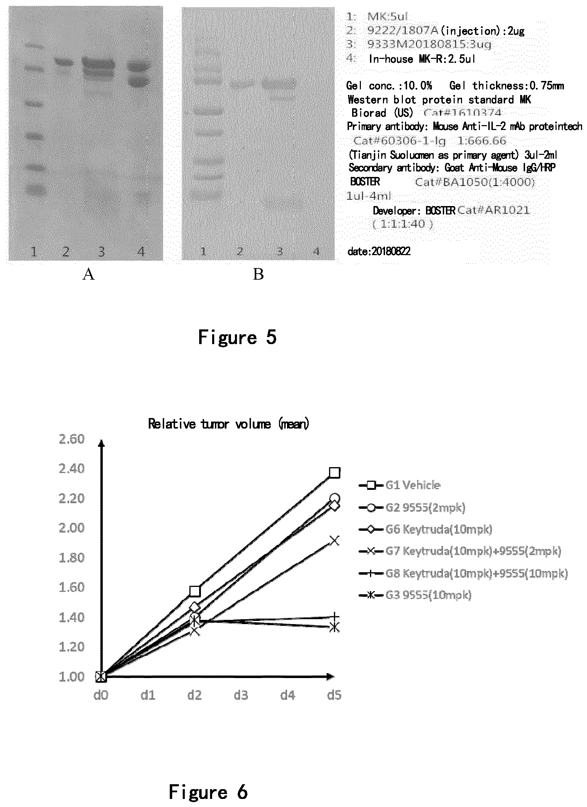

The recombinant human serum albumin/interleukin-2 fusion protein and its analog fusion proteins expressed and secreted by yeast were detected with an anti-human HSA mouse monoclonal antibody (Sigma Cat#: A6684), and confirmed to contain the protein sequence of human serum albumin. Similarly, Western blot experiments with an anti-human IL-2 rabbit monoclonal antibody (Abcam, US; Cat#: ab92381) after the same sample was subjected to electrophoresis and glue transfer showed that the fusion proteins had the same antigenicity as human IL-2. Therefore, it was confirmed that the expressed rhIL-2/SA fusion protein had the antigenicity of both human serum albumin and human interleukin-2 variants. Figure 5 shows an SDS-PAGE electropherogram (A) and Western-blot (B) of the recombinant human serum albumin/interleukin-2 fusion protein and its analog fusion proteins, showing that the fusion proteins have a significant immune reaction with specific antibodies against human interleukin-2. The standard molecular weights of the proteins used in SDS-PAGE were 97.4 kD, 66.2 kD, 43 kD, 31 kD, 20.1 kD and 14.4 kD from top to bottom, respectively; and the standard molecular weights of the proteins used in Western-blot were 250 kD, 150 kD, 100 kD, 75 kD, 50 kD, 37 kD, 25 kD, 20 kD, 15 kD and 10 kD from top to bottom, respectively (Bio-RAD). The results showed that the fusion protein and its analog fusion proteins all had significant immunoreactivity with anti-human interleukin-2 specific antibodies.

### Example 10: Determination of biological activity of recombinant human serum albumin/interleukin-2 fusion protein and its analog fusion proteins

According to General Rule 3524 of the 2015 edition of Chinese Pharmacopoeia (part III), CTLL-2 cell /MTT colorimetry was carried out for determining the biological activity of recombinant human interleukin-2. Based on the fact that at different concentrations of interleukin-2 (IL-2), the survival rate of the IL-2-dependent cell line CTLL-2 cells is different, the biological activity of IL-2 was detected.

Preparation of standard solution: The national standard for the determination of the biological activity of recombinant human interleukin-2 was reconstituted following the instructions for use, and then diluted with the basic culture solution to 200 IU/ml. In a 96-well cell culture plate, 2-fold serial dilutions were performed for a total of 8 dilutions, 2 wells for each dilution. 50 µl of standard solution was left in each well, and the excess solution was discarded. The above operations were carried out under aseptic conditions.

Preparation of test solution: The test sample was at reconstituted at an indicated amount, and then diluted with the basic culture solution to about 200 IU/ml. In a 96-well cell culture plate, 2-fold serial dilutions were performed for a total of 8 dilutions, 2 wells for each dilution. 50 µl of test solution was left in each well, and the excess solution was discarded. The above operations were carried out under aseptic conditions.

Determination method: CTLL-2 cells were cultured with the complete culture solution at 37°C and 5% CO₂ to a sufficient amount. They were collected by centrifugation, washed three times with the RPMI1640 culture solution, and then resuspended in the basic culture solution to prepare a cell suspension containing 6.0 × 10⁵ cells per ml, which was used for standby at 37°C and 5% CO₂. In the 96-well cell culture plate containing the standard solution and the test solution, 50 µl of cell suspension was added to each well, and the mixture was cultured at 37°C and 5% CO₂ for 18-24 hours. Then, 20 µl of MTT solution was added to each well, and the mixture was cultured at 37°C and 5% CO₂ for 4-6 hours. Subsequently, 150 µl of lysis buffer was added to each well, and the mixture was incubated at 37°C and 5% CO₂ for 18-24 hours. The above operations were carried out under aseptic conditions. The solution in the cell plate was mixed well, and put into a microplate reader, in which the absorbance was measured at the wavelength of 570 nm with 630 nm as the reference wavelength, and the measurement results were recorded.

The experimental data was processed by a computer program or the four-parameter regression calculation method, and the results were calculated according to the following formula:
biological activity of test sample (IU/ml) = Pr × (Ds × Es)/(Dr × Er)
in the formula, Pr is the biological activity of the standard, IU/ml; Ds is the pre-dilution factor of the test sample; Dr is the pre-dilution factor of the standard; Es is the dilution factor of the test sample equivalent to the median effective amount of the standard; and Er is the dilution factor of the median effective amount of the standard. Note: For the developing method, other developing methods that have been verified equivalently can also be used.

The finished product detection results of the recombinant human serum albumin/interleukin-2 fusion protein and its analog fusion proteins [rHSA/IL-2 (9222) and rhIL-2/SA (9333)] carried out by Jiangsu Kingsley Pharmaceutical Co., Ltd. showed that the specific bioactivity of the recombinant human serum albumin/interleukin-2 fusion protein (production batch number: 9222/1807A) was 2.46 × 10⁶ IU/mg, and the specific bioactivity of the recombinant human interleukin-2/serum albumin fusion protein (production batch number: 9333/1809A) was 2.39 × 10⁶ IU/mg. The results showed that the biological activity of interleukin-2 in the fusion proteins was not affected regardless of whether wild-type human interleukin-2 was located at the C-terminus or the N-terminus of the human serum albumin mature peptide. In other words, the site where interleukin-2 binds to the cell surface receptor was not interfered with by human serum albumin. The specific bioactivity of the stock batch 9555Y20190306 of the recombinant human serum albumin/interleukin-2 analog fusion proteins was determined to be about 1.49 × 10⁶ IU/mg at the cell level by using the method provided in Chinese Pharmacopoeia. This result showed that the specific bioactivity of the variant fusion proteins or analog fusion proteins, which were subjected to amino acid substitution, was significantly reduced at the cell level. In view of that the molecular weight of the recombinant human serum albumin/interleukin-2 fusion protein and its analog fusion proteins was 81.6 kD, and the molecular weight of the human IL-2 variant was 15.1 kD, the molecular weight of the IL-2 variant should account for 18.5% of the total fusion protein. The specific bioactivity value of recombinant human interleukin-2 (monomer) at the cell level is specified as 1 × 10⁷ IU/mg in Chinese Pharmacopoeia. The theoretical specific bioactivity of the fusion protein should be 0.15 × 10⁷ IU/mg in terms of theoretical molecular moles. The actual measurement result was exactly 0.149 × 10⁷ IU/mg, from which it can be seen that it is also possible whether the detection results are slightly biased under different detection conditions. In summary, it can be preliminarily determined that the IL-2 biological activity in the fusion proteins was not significantly reduced by fusion with albumin. The table below shows results of biological activity detection for a commercial recombinant human interleukin-2 (rhIL-2) product and four recombinant human serum albumin/interleukin-2 fusion protein and its analog fusion proteins.

| **Comparison of the biological activity of various fusion proteins of interleukin-2 and human serum albumin and their analog fusion proteins** | | | |
|---|---|---|---|
| Batch number | Product specification | Dilution factor | Specific bioactivity (IU/mg) |
| 9222Y20180701 | 1 mg/ml | 6000 | 1.76 × 10⁶ |
| 9333Y20180401 | 1mg/ml | 6000 | 0.98 × 10⁶ |
| 9555X20190306 | 1.04mg/ml | 6000 | 1.49 × 10⁶ |
| 9777B20190405 | 1.36mg/ml | 6000 | 2.15 × 10⁶ |
| Finished recombinant human interleukin-2 product (Jiangsu Kingsley Pharmaceutical Co., Ltd.) | 1.00 × 10⁶ IU/ml/bottle | 6000 | 1.32 × 10⁶ IU/ml |

### Example 11: Determination of in vivo half-life of recombinant human serum albumin/interleukin-2 fusion protein and its analog fusion proteins in rats

Sprague-Dawley rats (SD, SPF grade, half male and half female) were used. The rats were weighed on the day before administration (D-1), wherein the body weight of the male rats was in the range of 276-372 g, and the body weight of the female rats was in the range of 221-280 g. The positive control drug was recombinant human interleukin-2 (rhIL-2) stock (from Shandong Jintai Biopharmaceutical Co., Ltd.). The test drugs were the injections of the recombinant human serum albumin/interleukin-2 fusion protein and its analog fusion proteins of the present invention, and the specifications were all 2.5 mg/0.5 ml/bottle (the protein concentration was 5 mg/ml). The rats (three animals in each group) were administered at a dosing dose of 200 µg/kg based on that the content of rhIL-2 per mg of protein in the fusion proteins was only 1/4. The dosing volume for each rat was 300 µl. Blood samples were collected 1 h before administration, and 2 min, 0.5 h, 2 h, 4 h, 6 h, 8 h, 24 h, 48 h, 72 h, 96 h, 120 h and 168 h after administration. After the rats were anesthetized by inhalation of isoflurane, blood samples were collected from the capillaries of the orbital venous plexus at a blood collection volume of about 0.02-0.05 mL for each rat at every time. The collected blood samples were transferred into an EDTA-containing EP tube, shaken well, placed in crushed ice (stored in crushed ice for no more than 2 h), and then centrifuged at 4000 rpm at a low temperature (about 4°C) for 10 min to separate plasma, which was stored below -20°C for analysis.

The Human IL-2 Quantikine ELISA Kit (Cat#2050) produced by R&D Systems (US) was used. The blood samples were detected using the ELISA kit. The dilution of the blood samples for IL-2 was about 100-300 times that of the blood samples for the fusion proteins. In short, the measured absorption value of the blood samples at OD₄₅₀ should be between 0-4. Figure 6 shows a graph of plasma concentration determination and half-life determination of the recombinant human serum albumin/interleukin-2 fusion protein and its analog fusion proteins in rats after subcutaneous administration. The results showed that the lowest and highest blood half-life values of IL-2 measured in the three animals were 2.5 h and 4.5 h, respectively. In contrast, the lowest and highest blood half-life values of each of the recombinant human serum albumin/interleukin-2 fusion protein (9222, rHSA/IL-2), recombinant human interleukin-2/serum albumin fusion protein (9333, rhIL-2/SA) and recombinant human interleukin-2v5/serum albumin fusion protein (9555, rhIL-2v5/SA) of the present invention measured in the three animals were 68 h and 96 h, respectively (Figure 6). Taking the lowest blood half-life value 68 h of the fusion proteins as an example for calculation, the fusion proteins had long-acting effects about 15.1 times that of the positive control, when the half-life of the interleukin-2 monomer was 4.5 h; or about 27,2 times that of the positive control, when the half-life of the IL-2 monomer was 2.5 h. It is precisely because the recombinant human serum albumin/interleukin-2 fusion protein and its analog fusion proteins all had a significant and unexpectedly long metabolic cycle in animals, i.e., a long blood half-life, that interleukin-2 and its variants on the fusion proteins were slowly released. When used in combination with monoclonal antibody drugs, the fusion proteins with long-acting effects will have better application prospects and bring unexpected clinical treatment advantages.

### Example 12: Pharmacodynamic study in humanized mouse BALB/c-hPD1 model subcutaneously inoculated with CT26.WT colon cancer tumors at immunodetection site (tested by GemPharmatech Co., Ltd.)

The PD1 humanized mouse model independently developed by GemPharmatech Co., Ltd. is a mouse BALB/c-hPD1 model humanized with the programmed cell death receptor PD1. The establishment method is to replace the extracellular portion of PD1 of BALB/c mice with the corresponding human-derived fragment so as to completely retain the intracellular portion of the mouse PD1, thus obtain transgenic PD-1 humanized mice. Then, the transplanted tumor mouse model is established by subcutaneous inoculation of mouse colon cancer cell line CT26.WT. This mouse model has been used to evaluate the anti-tumor effects of various drugs in combination with anti-human PD-1 antibody drugs.

The recombinant human serum albumin/interleukin-2 fusion protein and its analog fusion proteins (rIL-2v5/SA, product code: 9555; and rHSA/IL-2v7, product code: 9777) provided by the inventors as test drugs together with the anti-PD-1 antibody Keytruda purchased from MSD provided by GemPharmatech Co., Ltd. were administered in groups. The animals were divided into blank preparation (Vehicle) control mice (group G1); rhIL-2v5/SA alone low-dose group (2 mpk, group G2) and high-dose group (10 mpk, group G3); rHSA/IL-2v7 alone low-dose group (2 mpk, group G4) and high-dose group (10 mpk, group G5); Keytruda alone group (10 mpk, group G6); rIL-2v5/SA low-dose (2 mpk) + Keytruda (10 mpk) group (group G7) and high-dose (10 mpk) + Keytruda (10 mpk) group (group G8); and rHSA/IL-2v7 low-dose (2 mpk) + Keytruda (10 mpk) group (group G9) and high-dose (10 mpk) + Keytruda (10 mpk) group (group G10), to investigate the anti-tumor effects when used alone or in combination (combination).

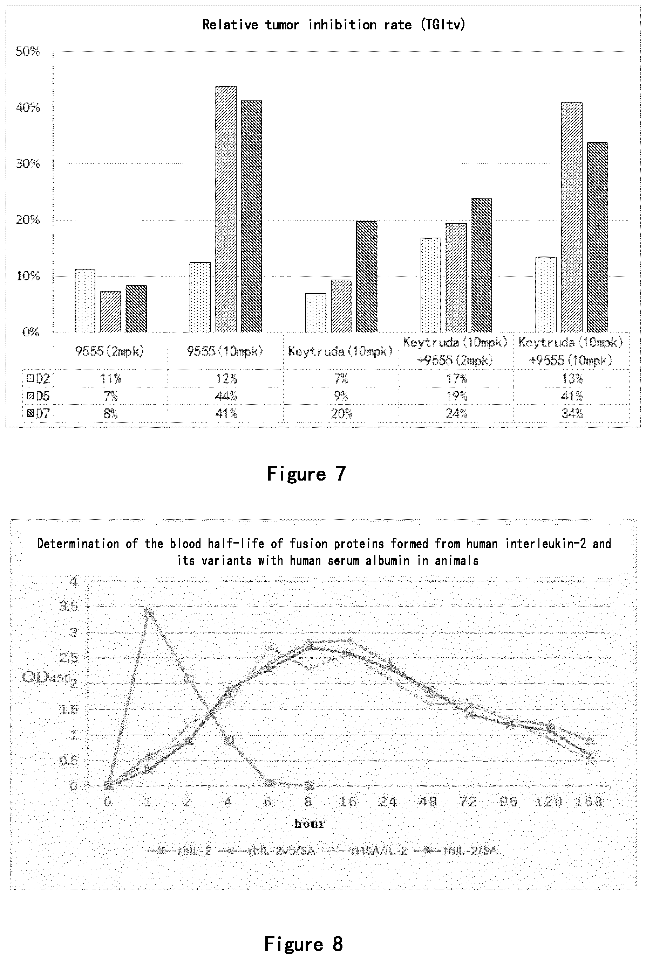

The results of the tumor growth inhibitory effects on Day 5 of the experiment were already very significant, see Figure 7, which shows relative changes in tumor volume between each administration group and the control group in the humanized PD-1 transgenic mouse tumor model after the administration of 9555 (molecular structure of the fusion protein: rIL-2v5/SA), one of the recombinant human serum albumin/interleukin-2 fusion protein and its analog fusion proteins. This showed that changes in tumor inhibition rate in group G3 - 9555 high-dose group (10 mpk), group G7 - 9555 low-dose (2 mpk) in combination with Keytruda (10 mpk) group, and group G8 - 9555 high-dose (10 mpk) in combination with Keytruda (10 mpk) group were all better than that in group G6 - Keytruda (10 mpk) alone group. The change in tumor inhibition rate in group G3 - 9555 high-dose group (10 mpk) was 43.80%, which was significantly different from that in the control group with P value = 0.035. As for the change in tumor volume in group G8 - 9555 high-dose (10 mpk) in combination with Keytruda (10 mpk) group (Figure 7), the change in tumor inhibition rate was 40.92%, which was significantly different from that in the control group with P value = 0.008 (Figure 8). It can be seen from the figure that the tumor inhibition rates in 9555 (one of the recombinant human serum albumin/interleukin-2 fusion protein and its analog fusion proteins of the present invention) alone low-dose group, the anti-PD-1 monoclonal antibody drug Keytrude alone group and Keytrude + 9555 (2 mg dose) group were 7.26%, 9.36% and 19.33% on Day 5, respectively. This result showed that the combination of Keytrude + rhIL-2v5/SA can improve the tumor inhibition ability of the antibody drug Keytrude by at least 1-fold or more, which had been significantly effective. Figure 8 shows a graph of efficacy results of the recombinant human serum albumin/interleukin-2 fusion protein analog 9555, alone or in combination with the anti-PD-1 antibody Keytruda, on days 2 (D2), 5 (D5) and 7 (D7) for the inhibition of the proliferation of mouse solid tumors.

One of the recombinant human serum albumin/interleukin-2 fusion protein and its analog fusion proteins (rHSA/IL-2v7, product code: 9777) provided by the inventors as the test drug together with the anti-PD-1 antibody Keytruda purchased from MSD provided by GemPharmatech Co., Ltd. were administered in groups. The results of tumor inhibition obtained in 5 days were different from those of 9555, see Figure 9, which shows relative changes in tumor volume between each administration group and the control group in the humanized PD-1 transgenic mouse tumor model after the administration of 9777 (molecular structure of the fusion protein: rHSA/IL-2v7), one of the recombinant human serum albumin/interleukin-2 fusion protein and its analog fusion proteins. Moreover, the combination of 9777 and Keytruda also showed increased efficacy for the inhibition of the proliferation of tumors in terms of synergism, although the effect was not as significant as the analog fusion protein with the molecular structure of 9555. However, further studies are needed to carry out to confirm whether there is a better efficacy after long-term administration.

After administration for a short time, the recombinant human serum albumin/interleukin-2 fusion protein and its analog fusion proteins alone can show a significant therapeutic effect of inhibiting the proliferation of solid tumors. When used in combination with anti-PD-1 antibodies, they can significantly improve the effect of inhibiting the proliferation of solid tumors.

To this end, the inventors stopped the administration of rhIL-2v5/SA (9555), but continued the administration of Keytrude at the same dose as the monotherapy. The results showed that after 3 days of drug withdrawal, that is, on Day 8 of the total experiment, the tumor growth inhibition rate in the Keytrude 10 mg + 9555 2 mg group was improved to 23.72%, while for the 9555 (2 mg) group that continued to be administered at the same time, it was 8.36%, and for the Keytrude alone (10 mg) group, it was 19.71%, still showing the synergistic effect of the combination. When used in combination in the high-dose group, even if rhIL-2/SA administration was stopped, the combination still achieved a tumor growth inhibition rate of 33.71%, which improved the efficacy by at least 1-fold. Clinically, if the evaluation point of tumor inhibition rate of 30% is achieved, it means that the therapeutic effect is clinically significant. Animal pharmacodynamic studies have shown that the fusion proteins of the invention may have significant clinical advantages.

A tumor growth inhibition rate of 40.92% was achieved in group G8 - one of the recombinant human serum albumin/interleukin-2 fusion protein and its analog fusion proteins 10 mg + Keytrude (10 mg) group on Day 5, while a striking tumor growth inhibition rate of 43.8% was also achieved in the rhIL-2v5/SA alone high-dose (10 mg) group on Day 5. The results showed that high doses of the fusion proteins of the present invention had a significant therapeutic effect, and when used in combination with antibodies, the combination did not result in a higher tumor growth inhibition rate. When the inventors stopped the administration of rhIL-2v5/SA and continued the administration of Keytrude, the tumors of all mice in this group immediately began to grow significantly, see Figure 10, which shows the tumor growth inhibitory effects between each administration group and the control group in the humanized PD-1 transgenic mouse tumor model after the administration of the recombinant human serum albumin/interleukin-2 fusion protein and its analog fusion proteins, especially the change of tumor proliferation after drug withdrawal. In Figure 10, the left panel shows the tumor growth 5 days after administration (tumor growth was severely inhibited). In Figure 10, the right panel shows the tumor growth in mice during days 5-8 after 3 days of drug withdrawal, showing that in the absence of drugs for inhibition, the tumors grew and developed rapidly again. The performance of the tumor growth with anti-tumor drugs for inhibition or drug withdrawal was consistent in all administration groups of mice. This experiment showed that the recombinant fusion proteins of the present invention had a particularly unexpected and significant effect in inhibiting tumor growth and a strong tumor growth inhibitory function. The long-acting recombinant interleukin-2 variant fusion proteins disclosed by the present invention, whether used alone or in combination with the anti-PD-1 antibody Keytrude, has the effect of strongly inhibiting tumor growth in mice, as well as a significant synergistic effect on anti-PD-1 antibodies. Furthermore, a very significant reduction in the phenomenon of cytokine storm was observed in the experiment.

### Example 13: Pharmacodynamic evaluation of tumor TILs in humanized PD-1 transgenic mouse model subcutaneously transplanted with colon cancer CT26 cells by flow cytometry

In order to test the recombinant human serum albumin/interleukin-2 fusion protein and its analog fusion proteins of the present invention, alone or in combination with Keytrude (an anti-PD-1 antibody), in the evaluation of the efficacy in subcutaneous tumor-bearing mice of the humanized mouse BALB/c-hPD1 model subcutaneously inoculated with CT26.WT colon cancer tumors at an immunodetection site, TIL cells in tumor tissues were detected by flow cytometry to observe the effects of the drugs on the immune system. Sample: tumors. Detection markers: CD45, CD3, CD8, CD4, CD49b, CD25 and FOXP3. The main instruments used were Thermo AttuneNxT flow cytometer, Eppendorf 5804R refrigerated centrifuge, tissue processor (GentleMACS), etc. Immune cells were enriched by centrifugation using a 40% + 70% discontinuous density gradient Percoll. Dead cells were labeled according to the instructions for use of FVS780. After washing twice with buffer, the cells were subjected to extracellular staining. The antibody mixture was prepared, 50 uL of antibody mixture was added to the samples in each tube, and the resultant was incubated at 4°C for 40-60 min in the dark. According to the instructions for use of Fix&Perm reagent 00-5523 (Ebioscience), anti-FOXP3 antibody was incubated at 4°C for 30 min in the dark after fixation and permeabilization, washed twice, and loaded onto the flow cytometer for detection. The main antibodies used were:

| **Marker** | **Dye** | **Supplier** | **Product catalog No.** |
|---|---|---|---|
| mCD45 | BV510 | BD | 563891 |
| mCD3 | AF700 | BD | 561388 |
| mCD4 | FITC | BD | 553729 |
| mCD8 | percp-cv5.5 | BD | 551162 |
| mCD49b | APC | ebioscience | 17-5971-81 |
| mCD25 | BV421 | BD | 564370 |
| mFOXP3 | PE | ebioscience | 12-5773-82 |
| Live/dead | FVS780 | BD | 565388 |

Figure 11 shows that the recombinant human serum albumin/interleukin-2 fusion protein and its analog fusion proteins of the present invention selectively activate and differentiate CD8+ cells and inhibit Treg cells obviously, which results are very similar to those of NKTR-214 (2019 ASCO). The G2 group (recombinant human interleukin-2v5/serum albumin fusion protein, rhIL-2v5/SA, code: 9555, in which G2 represented the dose group at a dosing dose of 2 mg) indicated by the dashed circle in Figure 11 showed a significant promotion effect on the proliferation of CD8+ cells, whereas it showed a tendency to significantly inhibit proliferation and decline in Treg cells. The increase of Treg cells is the key factor leading to the occurrence of cytokine storm. In the experiment, it was observed that the occurrence of cytokine storm in mice had indeed been significantly reduced. Studies have shown that when used in combination with anti-PD-1/L1 antibodies in anti-tumor drugs, the recombinant human serum albumin/interleukin-2 fusion protein and its analog fusion proteins can significantly improve the efficacy of the antibodies by 5-fold or more when compared with the antibodies alone. They likely showed a synergistic effect of at least 1-fold, 2-fold, 5-fold and 10-fold, and can also be used to prepare drugs for tumor treatment.

For the name of the test sample and the description of the dosing dose represented by each of the G1, G2, G4, G6, G7, G8 and G9 groups in Figure 11, see Example 12 for details.

The present invention shows that as long-acting drugs, the invented recombinant human serum albumin/interleukin-2 fusion protein and its analog fusion proteins have unexpected advantages over the existing commercially available interleukin-2 products, or PEG-interleukin-2 and Fc-interleukin-2 drugs in research at home and abroad. This result also fully supports that as drugs, the recombinant human serum albumin/interleukin-2 fusion protein and its analog fusion proteins prepared by the production process of the present invention, when used alone or in combination with anti-PD-1/L-1 antibodies, can be expected to meet the special needs of clinical treatment that the administration frequency is once every 2 weeks, or every 3 weeks, or even every 4 weeks, and have a significant synergistic effect on the clinical efficacy of the anti-PD-1 antibody drugs.

The present invention relates to the following embodiments.
1. A fusion protein, comprising a human serum albumin mature peptide and a human interleukin-2 mature peptide.
2. The fusion protein of embodiment 1, wherein the human serum albumin mature peptide and the human interleukin-2 mature peptide are linked directly.
3. The fusion protein of embodiment 1, wherein the human serum albumin mature peptide and the human interleukin-2 mature peptide are linked indirectly via a peptide linker.
4. The fusion protein of any one of embodiments 1-3, wherein the human interleukin-2 mature peptide is fused to the N-terminus of the human serum albumin mature peptide at its C-terminus.
5. The fusion protein of any one of embodiments 1-3, wherein the human interleukin-2 mature peptide is fused to the C-terminus of the human serum albumin mature peptide at its N-terminus.
6. The fusion protein of any one of embodiments 1-5, wherein the human serum albumin mature peptide has a wild-type human serum albumin mature peptide sequence.
7. The fusion protein of embodiment 6, wherein the wild-type human serum albumin mature peptide sequence is as set forth in SEQ ID NO. 9.
8. The fusion protein of any one of embodiments 1-5, wherein the human serum albumin mature peptide has a mutant human serum albumin mature peptide sequence.
9. The fusion protein of any one of embodiments 1-8, wherein the human interleukin-2 mature peptide has a wild-type human interleukin-2 mature peptide sequence.
10. The fusion protein of embodiment 9, wherein the wild-type human interleukin-2 mature peptide sequence is as set forth in SEQ ID NO. 10.
11. The fusion protein of any one of embodiments 1-8, wherein the human interleukin-2 mature peptide has a mutant human interleukin-2 mature peptide sequence.
12. The fusion protein of embodiment 11, wherein the mutant human interleukin-2 mature peptide sequence comprises one or more of the following substitutions relative to the wild-type human interleukin-2 mature peptide sequence: T3A, R38A, F42A, Y45A, E62A, L72G and C125A.
13. The fusion protein of embodiment 12, wherein the mutant human interleukin-2 mature peptide sequence comprises substitutions T3A, F42A, Y45A, L72G and C125A relative to the wild-type human interleukin-2 mature peptide sequence as set forth in SEQ ID NO. 10.
14. The fusion protein of embodiment 12, wherein the mutant human interleukin-2 mature peptide sequence comprises substitutions T3A, R38A, F42A, Y45A, E62A, L72G and C125A relative to the wild-type human interleukin-2 mature peptide sequence as set forth in SEQ ID NO. 10.
15. The fusion protein of embodiment 1, wherein a mutant human interleukin-2 mature peptide comprising substitutions T3A, F42A, Y45A, L72G and C125A relative to wild-type human interleukin-2 mature peptide is linked directly to the N-terminus of the human serum albumin mature peptide at its C-terminus.
16. The fusion protein of embodiment 1, wherein a mutant human interleukin-2 mature peptide comprising substitutions T3A, R38A, F42A, Y45A, E62A, L72G and C125A relative to wild-type human interleukin-2 mature peptide is linked directly to the C-terminus of the human serum albumin mature peptide at its N-terminus.
17. The fusion protein of embodiment 1, having an amino acid sequence selected from the group consisting of SEQ ID NO. 1, SEQ ID NO. 3, SEQ ID NO. 5 or SEQ ID NO. 7.
18. The fusion protein of any one of embodiments 1-17, which has reduced binding to human IL-2 receptor alpha (CD25) as compared to wild-type human interleukin-2.
19. The fusion protein of any one of embodiments 1-17, which does not bind to the human IL-2 receptor alpha (CD25).
20. The fusion protein of any one of embodiments 1-17, which has enhanced binding to IL-2 receptor beta (CD122) and IL-2 receptor gamma (CD132) as compared to wild-type human interleukin-2.
21. The fusion protein of any one of embodiments 1-17, which has an *in vivo* half-life that is extended at least 1-fold, at least 2-fold, at least 5-fold, at least 10-fold, at least 15-fold, at least 20-fold or more as compared to the wild-type human interleukin-2 or an amino acid sequence variant thereof.
22. The fusion protein of any one of embodiments 1-17, which has reduced cytotoxicity as compared to the wild-type human interleukin-2.
23. The fusion protein of any one of embodiments 1-17, which has increased activity in inhibiting tumor growth as compared to the wild-type human interleukin-2.
24. The fusion protein of any one of embodiments 1-17, which increases the activity of an anti-PD-1 antibody or an anti-PD-L1 antibody in inhibiting tumor growth by at least 1-fold, at least 2-fold, at least 5-fold, at least 10-fold, at least 15-fold, at least 20-fold, at least 25-fold, at least 50-fold, at least 100-fold or more.
25. The fusion protein of any one of embodiments 1-17, for use as a medicament.
26. The fusion protein of any one of embodiments 1-17, for use in the treatment of a tumor.
27. The fusion protein of any one of embodiments 1-17, for use in the treatment of a cancer.
28. The fusion protein of any one of embodiments 1-17, for use in the treatment of cancerous ascites.
29. A nucleic acid encoding the fusion protein of any one of embodiments 1-17.
30. The nucleic acid of embodiment 29, having a nucleotide sequence selected from the group consisting of SEQ ID NO. 2, SEQ ID NO. 4, SEQ ID NO. 6 or SEQ ID NO. 8.
31. A vector, comprising the nucleic acid of embodiment 29 or 30.
32. The vector of embodiment 31, which is a cloning vector or an expression vector.
33. The vector of embodiment 31 or 32, which is a plasmid vector or a viral vector.
34. A host cell, comprising the nucleic acid of embodiment 29 or 30, or comprising the vector of any one of embodiments 31-33.
35. The host cell of embodiment 34, which is a bacterial cell, a fungal cell, a plant cell or an animal cell.
36. The host cell of embodiment 34, which is an *E. coli* cell, a *Pichia pastoris* cell or a CHO cell.
37. A method of producing the fusion protein of any one of embodiments 1-17, comprising culturing the host cell of any one of embodiments 34-36, such that the fusion protein is produced.
38. A pharmaceutical composition, comprising the fusion protein of any one of embodiments 1-17.
39. Use of the fusion protein of any one of embodiments 1-17 in the preparation of a medicament for the treatment of a tumor.
40. Use of the fusion protein of any one of embodiments 1-17 in the preparation of a medicament for the treatment of a cancer.
41. Use of the fusion protein of any one of embodiments 1-17 in the preparation of a medicament for the treatment of cancerous ascites.
42. A method of treating a tumor, comprising administering the fusion protein of any one of embodiments 1-17 to a subject having the tumor.
43. A method of treating a cancer, comprising administering the fusion protein of any one of embodiments 1-17 to a subject having the cancer.
44. A method of treating cancerous ascites, comprising administering the fusion protein of any one of embodiments 1-17 to a subject having cancerous ascites.
45. The method of any one of embodiments 42-44, further comprising administering an anti-PD-1 antibody or an anti-PD-L1 antibody to the subject.
46. The method of any one of embodiments 42-45, wherein the fusion protein is administered at most once a week, once every two weeks, once every three weeks, once every four weeks or once a month.

## Claims

1. A fusion protein, comprising a human serum albumin mature peptide and a human interleukin-2 mature peptide,
wherein the human serum albumin mature peptide and the human interleukin-2 mature peptide are linked directly or indirectly via a peptide linker,
wherein the human interleukin-2 mature peptide is fused to the N-terminus of the human serum albumin mature peptide at its C-terminus, or the human interleukin-2 mature peptide is fused to the C-terminus of the human serum albumin mature peptide at its N-terminus,
wherein the human interleukin-2 mature peptide has a wild-type human interleukin-2 mature peptide sequence or a mutant human interleukin-2 mature peptide sequence comprising one or more of the following substitutions relative to the wild-type human interleukin-2 mature peptide sequence: T3A, R38A, F42A, Y45A, E62A, L72G and C125A, wherein the wild-type human interleukin-2 mature peptide sequence is optionally as set forth in SEQ ID NO. 10, and
wherein the human serum albumin mature peptide has a wild-type human serum albumin mature peptide sequence, optionally as set forth in SEQ ID NO. 9, or the human serum albumin mature peptide has a mutant human serum albumin mature peptide sequence.

2. The fusion protein of claim 1, wherein
(a) the mutant human interleukin-2 mature peptide sequence comprises substitutions T3A, F42A, Y45A, L72G and C125A relative to the wild-type human interleukin-2 mature peptide sequence, and is linked directly to the N-terminus of the human serum albumin mature peptide at its C-terminus; or
(b) the mutant human interleukin-2 mature peptide sequence comprises substitutions T3A, R38A, F42A, Y45A, E62A, L72G and C125A relative to the wild-type human interleukin-2 mature peptide sequence, and is linked directly to the C-terminus of the human serum albumin mature peptide at its N-terminus.

3. The fusion protein of claim 1, having the amino acid sequence of SEQ ID NO. 1, SEQ ID NO. 3, SEQ ID NO. 5 or SEQ ID NO. 7.

4. The fusion protein of any one of claims 1-3, having one or more of the following properties:
(a) it has reduced binding to human IL-2 receptor alpha (CD25) as compared to wild-type human interleukin-2;
(b) it does not bind to the human IL-2 receptor alpha (CD25);
(c) it has enhanced binding to IL-2 receptor beta (CD122) and IL-2 receptor gamma (CD132) as compared to wild-type human interleukin-2;
(d) it has an *in vivo* half-life that is extended at least 1-fold, at least 2-fold, at least 5-fold, at least 10-fold, at least 15-fold, at least 20-fold or more as compared to the wild-type human interleukin-2 or an amino acid sequence variant thereof;
(e) it has reduced cytotoxicity as compared to the wild-type human interleukin-2;
(f) it has increased activity in inhibiting tumor growth as compared to the wild-type human interleukin-2; and/or
(g) it increases the activity of an anti-PD-1 antibody or an anti-PD-L1 antibody in inhibiting tumor growth by at least 1-fold, at least 2-fold, at least 5-fold, at least 10-fold, at least 15-fold, at least 20-fold, at least 25-fold, at least 50-fold, at least 100-fold or more.

5. A nucleic acid encoding the fusion protein of any one of claims 1-4, optionally having the nucleotide sequence of SEQ ID NO. 2, SEQ ID NO. 4, SEQ ID NO. 6 or SEQ ID NO. 8.

6. A host cell comprising the nucleic acid of claim 5, optionally a bacterial cell, a fungal cell, a plant cell or an animal cell, further optionally a *Pichia pastoris* cell or a CHO cell.

7. A method of producing the fusion protein of any one of claims 1-4, comprising culturing the host cell of claim 6, such that the fusion protein is produced.

8. A pharmaceutical composition, comprising the fusion protein of any one of claims 1-4.

9. Use of the fusion protein of any one of claims 1-4 in the preparation of a medicament for the treatment of a tumor, a cancer or cancerous ascites.

10. A method of treating a tumor, a cancer or cancerous ascites, comprising administering the fusion protein of any one of claims 1-4 to a subject having the tumor, the cancer or cancerous ascites, optionally further comprising administering an anti-PD-1 antibody or an anti-PD-L1 antibody to the subject.
